# EUROPEAN PATENT SPECIFICATION

(11) **EP 2 459 242 B1**
(45) Date of publication and mention of the grant of the patent: **21.10.2015**
(21) Application number: 10739478.5
(22) Date of filing: 28.07.2010
(51) Int. Cl.: A61L 27/34, A61L 27/50, A61L 29/08, A61L 29/14

(54) **A MEDICAL DEVICE OF POLYOLEFIN**
MEDIZINISCHE VORRICHTUNG MIT EINEM POLYOLEFIN
DISPOSITIF MEDICAL COMPRENANT UNE POLYOLEFINE

(30) Priority: 31.07.2009 DK 200970076; 02.11.2009 DK 200970188
(43) Date of publication of application: 06.06.2012
(73) Proprietor: Coloplast A/S, 3050 Humlebæk (DK)
(72) Inventor: NIELSEN, Henrik, Lindenskov, DK-2765 Smoerum (DK)
(86) International application number: PCT/DK2010/050198
(87) International publication number: WO 2011/012137

(56) References cited:
- EP-A1- 1 688 470
- WO-A1-2006/037321
- WO-A1-2008/071796
- WO-A2-2008/012325

## Description

### FIELD OF THE INVENTION

The present invention relates to a method for the preparation of a medical device element selected from tubes and catheters by means of extrusion, injection moulding or powder coating. The invention further relates to medical devices comprising such extruded, injection moulded or powder coated medical device elements. The medical device elements are characterized by a prefabricated shaped article or a thermoplastic substrate polymer having thereon a layer of a covalently cross-linked coating composition of a hydrophilic polymer and a low molecular weight scaffold having a plurality of residues of photo-initiator moieties covalently linked thereto and/or covalently incorporated therein.

### BACKGROUND OF THE INVENTION

Many medical devices require a lubricated surface. In the medical field, simple devices such as, for example, catheters and guide wires, can be inserted into a body cavity or through the skin and at a later time be withdrawn. Patient treatment often includes catheterization procedures or nutrition delivery systems, most of which involve invasive techniques. In all such cases effective lubrication, which is stable throughout both the insertion and withdrawal stages of the procedure, contributes greatly to patient comfort.

US 5,084,315 discloses a method for preparing a shaped article, e.g. by co-extrusion, utilizing a composition including poly(ethylene oxide) (PEO) and a polyurethane, which are not covalently cross-linked. The surface of the article is said to be lubricious when contacted with water.

US 5,061,424 discloses a method for preparing a shaped article, e.g. by co-extrusion, utilizing a composition including poly(vinyl pyrrolidone) (PVP) and a polyurethane, which are not covalently cross-linked. The surface of the article is said to be lubricious when contacted with water.

US 6,447,835 discloses a method of preparing a coated hollow polymeric tubular member for a medical device by co-extruding the tube together with a coating. The coating may comprise poly(ethylene oxide). The coating may also comprise acrylic monomers which may be reacted to form a cross-linked acrylic polymer network after extrusion.

WO 2008/012325 A2 discloses a method for making thermoplastic coatings by cross-linking by means of UV-irradiation.

WO 2008/071796 A1 discloses a method for making medical devices with coatings prepared from poly(ethylene oxide) and photo-initiator-containing scaffolds by cross-linking by means of UV-irradiation. The coating compositions are extruded, injection molded or powder coated on a prefabricated shaped article and/or on a thermoplastic substrate polymer.

### SUMMARY OF THE INVENTION

Although the shaped articles of US 5,084,315 and US 5,061,424 may have certain desirable and - for some applications - satisfactory properties with respect to reduced friction, the present inventors have found that it was not possible to combine the exceptionally low friction required for certain medical devices, such as catheters and guide wires, with a sufficient cohesion of the coating and a sufficient adhesion of the coating to a substrate. Hence, the present inventors found it necessary to develop methods for the preparation of medical devices which provide advantages with respect to simplicity, exceptionally low friction, excellent cohesion and excellent adhesion.

The present invention provides an alternative route involving the application of a particular scaffold having a plurality of photo-initiator moieties covalently linked thereto and/or covalently incorporated therein. Hence, the present invention i.a. provides a method for the preparation of medical devices which provide advantages with respect to simplicity and which provide advantages with respect to exceptionally low friction, excellent cohesion and excellent adhesion.

Furthermore, the present invention is using a special polyolefin for making a coated multilayer catheter. The materials identified additionally gives good catheter performance for the users like good flexibility, good anti-kink properties and good vibration damping properties. The materials are especially suitable for multilayer UV cured thermoplastic catheter coatings.

### BRIEF DESCRIPTION OF THE DRAWING

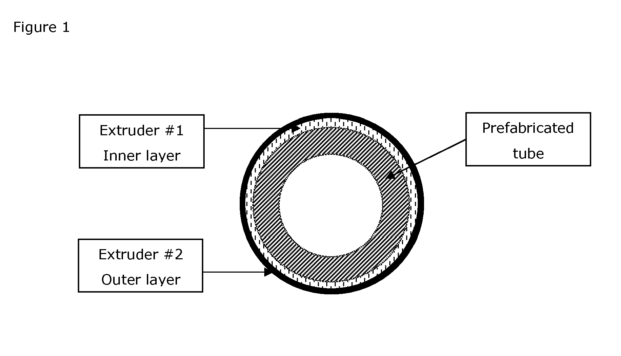

Figure 1 illustrates a medical device (e.g. a tube of catheter) of a prefabricated tube, a layer of a thermoplastic substrate polymer, and a covalently cross-linked coating composition.

### DETAILED DESCRIPTION OF THE INVENTION

### Abbreviations

| Trade name/ trivial name/ abbreviation | Chemical name |
|---|---|
| 2-BBCI | 2-Benzoylbenzoyl chloride |
| Bomar^{§)} | Aliphatic urethane oligomer with covalently bonded Irgacure 2959 UV functionalities |
| BTDA | 3,3',4,4'-Benzophenonetetracarboxylic acid dianhydride |
| Chivacure 3482 | 2-Methyl-1-[4-(alkylthio)phenyl]-2-(4-morpholinyl)-1-propanone (alkyl chain not revealed) |
| Chivacure 3690 | 2-Benzyl-2-(dimethylamino)-1-[4-(alkylmethylamino)phenyl]-1-butanone (alkyl chain not revealed) |
| CMC | Carboxymethylcellulose |
| Darocur 1173 | 2-Hydroxy-2-methylpropiophenone; 2-hydroxy-2-propyl phenyl ketone |
| Darocur TPO | Diphenyl(2,4,6-trimethylbenzoyl) phosphine oxide |
| DMAEMA | N,N-Dimethylaminoethyl methacrylate |
| DMF | N,N-Dimethylformamide |
| DMSO | Dimethylsulfoxide |
| EAA | Ethylene acrylic acid |
| EAA-EMA | Terpolymer of EAA and EMA |
| EEA | Copoly(ethylene/ethyl acrylate) |
| EMA | Copoly(ethylene/methyl acrylate) |
| EMAA | Ethylene methacrylic acid |
| EnBA | Copoly(ethylene/n-butyl acrylate) |
| Esacure KIP 150 | Oligo{2-hydroxy-2-methyl-1-[4-(1-methylvinyl)phenyl]propanone} |
| Esacure One | "Difunctional α-hydroxy ketone" (structure not revealed) |
| EVA | Copoly(ethylene/vinyl acetate) |
| EVA g-MAH | Copoly(ethylene/vinyl acetate)-graft-poly(maleic anhydride) |
| EVOH | Copoly(ethylene/vinyl alcohol) |
| GMA | Glycidyl methacrylate (2,3-epoxypropyl methacrylate) |
| HPEU | Hydrophilic polyetherurethane |
| Irgacure 127 | *Bis*(4-(2-hydroxy-2-propylcarbonyl)phenyl)methane |
| Irgacure 184 | 1-Hydroxy-1-cyclohexyl phenyl ketone |
| Irgacure 2959 | 2-Hydroxy-2-propyl 4-(hydroxyethoxy)phenyl ketone |
| Irgacure 369 | 2-Benzyl-2-(dimethylamino)-1-[4-(4-morpholinyl)phenyl]-1-butanone |
| Irgacure 379 | 2-(4-Methylbenzyl)-2-(dimethylamino)-1-[4-(4-morpholinyl)phenyl]-1-butanone |
| Irgacure 651 | Benzil α,α-dimethyl ketal; α,α-dimethoxy-α-phenylacetophenone; 2,2-dimethoxy-1,2-diphenyl-1-ethanone |
| Irgacure 819 | Phenyl*bis*(2,4,6-trimethylbenzoyl) phosphine oxide |
| Irgacure 907 | 2-Methyl-1-[4-(methylthio)phenyl]-2-(4-morpholinyl)-1-propanone |
| Irganox 1010 | Benzenepropanoic acid, 3,5-bis(1,1-dimethylethyl)-4-hydroxy-, 2,2-bis[[3-[3,5-bis(1,1-dimethylethyl)-4-hydroxyphenyl]-1-oxopropoxy]methyl]-1,3-propanediyl ester |
| LDPE | Low density polyethylene, e.g. Riblene LH10 from Polimeri |
| LLDPE | Linear low density polyethylene |
| MAH | Maleic anhydride |
| MDI | Methylene-4,4'-diphenyldiisocyanate |
| NMP | N-Methylpyrrolidone |
| NVP | N-Vinyl pyrrolidone |
| Omnipol BP | Poly(tetramethylene glycol) 250 diester of 4-benzoylphenoxyacetic acid |
| Omnipol TX | Poly(tetramethylene glycol) 250 diester of 2-thioxanthonyloxyacetic acid |
| PE | Polyethylene |
| PE g-MAH | Polyethylene-*graft*-poly(maleic anhydride) |
| Pebax | Polyether-*block*-polyamide |
| PEG | Poly(ethylene glycol) |
| PEG 2000 | Poly(ethylene glycol) with Mw 2000 g/mol |
| PEO | Poly(ethylene oxide) |
| PMDA | Pyromellitic acid dianhydride; 1,2,4,5-benzenetetracarboxylic acid dianhydride |
| PP | Polypropylene |
| PS | Polystyrene |
| PVC | Poly(vinyl chloride) |
| PVC, plasticized | Polyvinylchloride with plasticizer |
| PVOH | Poly(vinyl alcohol) |
| PVP | Poly(vinyl pyrrolidone) |
| PVP-DMAEMA | Copoly(vinyl pyrrolidone/N,N-dimethylaminoethyl methacrylate) |
| SBS | Polystyrene-*block*-polybutadiene-*block*-polystyrene |
| SEBS | Polystyrene-*block*-poly(ethylene/butylene)-*block*-polystyrene |
| SEEPS | Polystyrene-*block*-hydrogenated poly(isoprene/butadiene)-*block*-polystyrene |
| SEPS | Polystyrene-*block*-poly(ethylene/propylene)-*block*-polystyrene |
| SIS | Polystyrene-*block*-polyisoprene-*block*-polystyrene |
| SMA | Poly(styrene-*co*-maleic anhydride) |
| THF | Tetrahydrofuran |
| TPU | Thermoplastic polyurethane, e.g. Estane 58212 and Estane 58311 polyetherurethane from Lubrizol |
| VLDPE | Very low density polyethylene, e.g. Engage 8440 from Dow Ch. |

| | |
|---|---|
| ^{§}: See J.A. Leon, I.V. Khudyakov from Bomar Specialties, USA (2005): "UV-Light Sensitive (LSR) Urethane Acrylate Oligomers", Proceedings from RadTech Europe 05, Barcelona, Spain, October 18-20 2005, vol. 2, p. 359-64, Vincentz. | |

### The method of the invention

As mentioned above, the present invention relates to a method for the preparation of a medical device element, said method comprising the steps of:
(i) providing a prefabricated shaped article and/or one or more layers of a thermoplastic substrate polymer;
(ii) providing a coating composition comprising:
   (a) as the only polymer constituent(s), one or more hydrophilic polymer(s), and
   (b) one or more low molecular weight scaffolds having a plurality of photo-initiator moieties covalently linked thereto and/or covalently incorporated therein,
      wherein the photo-initiator moieties constitute 0.01-20 % by weight of the combined amount of the hydrophilic polymer(s) and the one or more low molecular weight scaffolds;
(iii) extruding, injection moulding or powder coating the coating composition of step (ii) on the prefabricated shaped article and/or the thermoplastic substrate polymer of step (i) so as to provide the medical device element of said prefabricated shaped article and/or said substrate polymer having thereon a layer of said coating composition, wherein, when both of said prefabricated shaped article and said substrate polymer are present, said prefabricated shaped article has thereon a layer of said substrate polymer;
(iv) irradiating the coating composition with UV or visible light so as to covalently cross-link said coating composition
wherein the prefabricated shaped article and/or one or more of the thermoplastic substrate polymers of step (i) comprising a polymer selected from the group of:
Ethylene (Meth)acrylic Acid copolymer
Ethylene - (Meth)acrylic Acid - Acrylic ester - terpolymer
Ethylene (Meth)acrylic Acid ionomer; and
wherein the medical device element is selected from tubes and catheters.

For medical tubes and catheters traditionally plasticized poly(vinyl chloride) (PVC) has been the material of choice due to its good flexibility, anti-kink properties and damping. During the last 20 years there has been more and more focus on substituting PVC with polyolefins and other materials like polyolefin thermoplastic elastomer (TPE) compounds and pure TPE's like thermoplastic polyurethane and polyether amide. These materials can give the desired flexibility, but the desired anti-kink properties and damping properties have been difficult to obtain. It has also been difficult to make a coating adhere to the polyolefin type of materials, and the price for all the materials has been rather high as they are either specially compounded polyolefin TPE's, often based on either styrene block copolymers like polystyrene-block-poly(ethylene/butylene)-block-polystyrene (SEBS), polystyrene-block-poly(ethylene/propylene)-block-polystyrene (SEPS) or polystyrene-block-polybutadiene-block-polystyrene (SBS) compounds or on rather expensive pure TPE's like thermoplastic polyurethane and polyether amide.

It has now surprisingly been found that tubes and catheters can be made of special polyolefin copolymers giving the above mentioned properties such as good anti-kink and damping properties. It is easy for a hydrophilic coating without using rather harmful organic solvents and/or harmful isocyanate or acrylic monomers to adhere to these materials.

In one embodiment of the present invention the coating of these tubes can be achieved by a tie-layer and a hydrophilic coating. The tube can either be monolayer or multilayer.

By tie-layer is meant one layer of a thermoplastic substrate polymer.

According to an embodiment of the invention the polyolefin polymers of choice for the tube layer(s) and tie-layer are from the group of:
Polyethylene acrylic acid copolymers (EAA) containing from 3% to 20% acrylic acid.
Polyethylene acrylic acid copolymers (EMAA) containing from 3% to 20% acrylic acid
Terpolymers of poly(ethylene/methyl acrylate) copolymer (EMA) or EMAA with acrylates like EMA, poly(ethylene/ethyl acrylate) copolymer (EEA) or poly(ethylene/butyl acrylate) copolymer (EBA).
Ionomers of EEA and EMAA with Li+, Na+, K+ or Zn+

In an embodiment of the invention a hydrophilic coated catheter multilayer tube consists of minimum one inner tube material, one tie-layer material and one outer hydrophilic coating material as provided.

In another embodiment the above mentioned materials can be combined with other materials. For instance an EAA tube can be used together with an EMA as the tie-layer and an EEA tube can be used together with an EMAA as the tie-layer.

Furthermore, the invention is based on the finding that cross-linking of the coating composition subsequent to extrusion, injection moulding or powder coating by means of one or more photo-initiator(s) and UV or visible light provides medical device elements which have good adhesion of the coating composition including the hydrophilic polymer to the prefabricated shaped article or the substrate polymer; good cohesion of the coating composition; and good water retention of the hydrophilic polymer in the wet state and thereby excellent properties with respect to low friction for an extended period of time.

The good properties with respect to good water retention of the hydrophilic polymer and excellent properties with respect to low friction for an extended period of time is somewhat contradictory to the fact that the flexibility of the polymer chains will be restricted by means of the cross-linking of the polymer and anchoring to the substrate polymer or prefabricated shaped article. Moreover, the presentation of the photo-initiator moieties as covalently linked to and/or covalently incorporated in a scaffold appears to further facilitate the above-mentioned useful properties.

### Medical device

The term "medical device" should be interpreted in a fairly broad sense. As examples of known medical devices (including instruments) may be mentioned catheters (such as urinary catheters), endoscopes, laryngoscopes, tubes for feeding, tubes for drainage, endotracheal tubes, guide wires, sutures, cannulas, needles, thermometers, condoms, urisheaths, barrier coatings e.g. for gloves, stents and other implants, contact lenses, extra corporeal blood conduits, membranes e.g. for dialysis, blood filters, devices for circulatory assistance, condoms, dressings for wound care, and ostomy bags. Most relevant are catheters, endoscopes, laryngoscopes, tubes for feeding, tubes for drainage, guide wires, sutures, and stents and other implants. Particularly interesting medical devices within the context of the present invention are catheters, such as urinary catheters.

Some medical devices may be constructed of one or more medical device elements which, when being assembled or rearranged, represent the ready-to-use medical device. Reference to a "medical device element" and "catheter element" means the medical device or catheter as such (i.e. one piece of medical device or catheter) or a part of a "ready-to-use" medical device or catheter.

Medical device elements selected from tubes and catheters are in the present context formed from a prefabricated shaped article and/or a thermoplastic substrate polymer and a coating composition. Upon (co)extrusion or injection moulding of the prefabricated shaped article and/or the thermoplastic substrate polymer and the simultaneous or subsequent application of the coating composition by co-extrusion, injection moulding or powder coating, at least a part of the surface of the prefabricated shaped article or the thermoplastic substrate polymer becomes coated with the coating composition as will be explained in more detail in the following. In some embodiments, the coating composition (i.e. a hydrophilic coating) is covering the full (outer) surface of the prefabricated shaped article/substrate polymer, and in some other embodiments, only to a part of the surface thereof. In the most relevant embodiments, the coating composition covers at least a part of the surface (preferably the whole surface) of the medical device that - upon proper use - comes into direct contact with body parts for which the medical device is intended.

### Prefabricated shaped articles

In the embodiments where a prefabricated shaped article is involved, the method is designed to provide a coating onto such shaped article. A wide variety of shaped articles are envisaged (e.g. tubes, catheters, guides, laparoscopic accessories, surgical instruments), just as a number of different materials may constitute such shaped articles, such as metals and alloys, e.g. stainless steel cores or typical guide-wire alloys, e.g. Ti alloys such as Nitinol and pseudoplastic Beta Ti-Mo-V-Nb-Al alloys.

Glasses and ceramics just as thermoplastic polymers are also envisaged. Suitable materials also include: Thermoplastic polymers such as hydrophilic polyurethanes, hydrophobic polyurethanes, polyether block amides (e.g. PebaxTM), PVC, polyamides, polyesters, biodegradable polyesters, polyacrylates, PS, silicones, latex rubber; block copolymers with the different structures diblock (A-B), multiblock (A-B)n or triblock (A-B-A) such as SEBS, SIS, SEPS, SBS, SEEPS (the block copolymers may be grafted with maleic anhydride onto the rubber block, typically the mid-block for triblock copolymers); thermoplastic polymers such as LDPE, LLDPE, VLDPE, PP, PE, and copolymers of ethylene and propylene, metallocene polymerized polyolefins, PS, EMA, EEA, EnBA, PE g-MAH, EVA, EVOH and vinyl acetate copolymer grafted with maleic anhydride (EVA g-MAH), or combinations thereof e.g. Orevac® ethylene-vinyl acetate-maleic anhydride terpolymers; and the functional polyolefins range, such as Lotader® ethylene-acrylic ester terpolymers with either MAH or GMA; and the maleic anhydride grafted polymers of PE, PP, PS, etc; and EAA, EMAA, Ethylene - (Meth)acrylic Acid - Acrylic ester - terpolymer, Ethylene (Meth)acrylic Acid ionomer with e.g. Na+, Li+, K+, Zn+.

It should be understood that the expression "(Meth)acrylic acid" is intended to encompass Methacrylic acid as well as Acrylic acid.

The abbreviations are explained in the above mentioned table.

### Thermoplastic substrate polymer

In the embodiments where a thermoplastic substrate polymer is involved, the method is designed to provide a coating onto this substrate. The thermoplastic substrate polymer is selected so as to provide the physical shape of the medical device element or so as to provide a suitable interface between the coating composition and the prefabricated shaped article.

According to one embodiment of the invention the thermoplastic substrate is consisting of more than one layer, preferably two layers, of a thermoplastic substrate polymer. The layers may be of different or identical type of polymers.

One layer of a thermoplastic substrate polymer may be used as a tie-layer.

Hence, the substrate polymer is typically selected from polyurethanes, polyether block amides (e.g. PebaxTM), PVC, polyamides, polyesters, polyacrylates, PS, silicones, latex rubber, SEBS, SIS, SEPS, SEEPS, EVA, PE, and copolymers of ethylene and propylene; thermoplastic polymers such as hydrophilic polyurethanes, hydrophobic polyurethanes, polyether block amides (e.g. PebaxTM), PVC, polyamides, polyesters, polyacrylates, PS, silicones, latex rubber; block copolymers with the different structures diblock (A-B), multiblock (A-B)n or triblock (A-B-A) such as SEBS, SIS, SEPS, SBS, SEEPS; the block copolymers maybe grafted with MAH onto the rubber block, typically the mid-block for triblock copolymers; thermoplastic polymers such as LDPE, LLDPE, VLDPE, PP, PE, and copolymers of ethylene and propylene, metallocene polymerized polyolefins, PS, EMA, EEA, EnBA, PE g-MAH, EVA, EVOH and EVA g-MAH, or combinations thereof, e.g. Orevac® ethylene-vinyl acetate-maleic anhydride terpolymers; the functional polyolefins range, such as Lotader® ethylene-acrylic ester terpolymers with either MAH or GMA; maleic anhydride grafted polymers of PE, PP, PS, etc.; and the EPOCROS K-series of reactive acrylate-oxazoline copolymers or the RPS/RAS-series of styrene-oxazoline copolymers, or styrene-acrylonitril-oxazoline copolymers; and EAA, EMAA, Ethylene - (Meth)acrylic Acid - Acrylic ester- terpolymer, Ethylene (Meth)acrylic Acid ionomer with e.g. Na+, Li+, K+, Zn+.

Currently very relevant materials for use as the thermoplastic substrate polymer are EAA, EMAA, Ethylene - (Meth)acrylic Acid - Acrylic ester - terpolymer, Ethylene (Meth)acrylic Acid ionomer with e.g. Na+, Li+, K+, Zn+.

### Coating composition

The principal constituents of the coating composition are the hydrophilic polymer(s) and the low molecular weight scaffold(s) having a plurality of photo-initiator moieties covalently linked thereto and/or covalently incorporated therein. These constituents will be discussed in detail further below.

Depending on the intended use, additives may be incorporated into the coating composition in order to achieve particular properties. For example one or more additives such as flow aids, flatting agents, heat stabilizers, surface cure modifiers, antibacterial agents, and osmolality increasing compounds may be added to the coating composition. Such additives and their use to modify polymer properties are conventional and well known to those skilled in the art. Such other components may be used in an amount of up to 10 % by weight, e.g. up to 5 % by weight, of the coating composition.

The antibacterial agent may be a silver salt, e.g. silver sulphadiazine; an acceptable iodine source such as povidone iodine (also called PVP iodine); chlorhexidine salts such as the gluconate, acetate, hydrochloride or the like; or salts or quaternary antibacterial agents such as benzalkonium chloride or other antiseptics or antibiotics. Antibacterial agents reduce the risk of infection, e.g. when urodynamic examinations are performed.

For medical devices or instruments suitable for introduction into human cavities it may be advantageous to include an osmolality increasing compound, e.g. a water-soluble non-ionic compound such as glucose, sorbitol, glycerin, or urea; or ionic compounds such as halides, nitrates, acetates, citrates or benzoates of alkali metals or alkaline earth metals or silver; or carboxylic acids such as acetic acid, etc.

It may further be desirable to include a plasticizer in the coating composition in order to facilitate the extrusion, injection moulding or powder coating. In such instances, a plasticizer may be included in an amount of up to 10 % by weight of the coating composition. Examples of such plasticizers include carboxylic acid-based plasticizers, such as partially esterified citric acids obtained from Jungbunzlauer and such citric acid esters obtained from Grindsted products, e.g. GRINDSTED-CITREM. It should be understood that plasticizers within the present context are generally to be understood as low-molecular weight constituents.

In one embodiment, the coating composition preferably consists of:
20-99.99 % by weight of the one or more hydrophilic polymer(s),
0-10 % by weight of one or more plasticizers,
0.01-80 % by weight of the one or more low molecular weight scaffolds, and
0-5 % by weight of other components.

In a more interesting embodiment, the coating composition consists of:
30-99.9 % by weight of the one or more hydrophilic polymer(s),
0-5 % by weight of one or more plasticizers,
0.1-70 % by weight of the one or more low molecular weight scaffolds, and
0-5 % by weight of other components.

In a particular embodiment, the coating composition consists of:
40-99 % by weight of the one or more hydrophilic polymer(s),
1-60 % by weight of the one or more low molecular weight scaffolds, and
0-5 % by weight of other components.

In another particular embodiment, the coating composition consists of:
50-99 % by weight of the one or more hydrophilic polymer(s),
0-10 % by weight of one or more plasticizers,
1-50 % by weight of the one or more low molecular weight scaffolds, and
0-5 % by weight of other components.

### Hydrophilic polymer

The main requirement to the hydrophilic polymer is to ensure that the covalently cross-linked coating composition becomes very slippery when it is swollen with hydrophilic liquids such as water or glycerol. Hence, the main function of the hydrophilic polymer(s) is to give the swollen coating low friction and high water retention.

The hydrophilic polymer is preferably also limpid at the temperature of photo-curing and has low absorbance in UV-C, UV-B and UV-A, so that it does not block the UV or visible light intended for the photo-initiator(s). The hydrophilic polymer may suitably be selected from one or more of the following materials:
- Poly(vinyl lactams) such as PVP; and copolymers of NVP and DMAEMA, (meth)acrylic acid, (meth)acrylic esters including 2-sulfoethyl methacrylate, (meth)acrylic amides including N,N-dimethylacrylamide and N-vinylacetamide, MAH, maleic esters, P- vinylphosphonic acid, methyl vinyl ether, etc.
- Slightly cross-linked PVP or PVP copolymers are preferred.
- Linear or, preferably, cross-linked PEO with high molecular weight, and copolymers of EO and PO.
- Superabsorbent homo- and copolymers of water-soluble alpha,beta-ethylenically unsaturated carboxylic acids and derivatives, such as acrylic acid, methacrylic acid, fumaric acid, maleic acid, crotonic acid, tiglic acid, and itaconic acid; and their esters and amides.
- Cellulosic superabsorbent polymers, e.g. hydroxypropyl methylcellulose or CMC, or starch-graft copolymers, such as starch-*graft*-polyacrylonitrile, starch *-graft-*poly(acrylic acid) and the like.
- PVOH, homo- and copolymers of sulphonic acid group containing monomers, such as S-vinylsulphonic acid, sodium sulfoethyl methacrylate, 2-acrylamido-2-methylpropane-sulphonic acid or the sodium salt (AMPS) and the like.
- *Alt*-copoly(methyl vinyl ether/maleic anhydride) (tradename Gantrez at ISP Corporation) which has been either hydrolyzed in basic solution (to form a polyanion), hydroxy-modified (to form an ester acid) or amino-modified (to form an amide acid).
- Poly(vinyl methyl ether), polyethyleneimine, poly(2-ethyl-2-oxazoline), copoly(2-ethyl-2-oxazoline/2-phenyl-2-oxazoline) as random or block copolymers, or the hydrophilic EPOCROS WS-series, such as WS-500, WS-700.
- Hydrophilic polyurethanes such as Tecogel 500 and Tecogel 2000 from Noveon, or Hydromed TP from Cardiotech.

The preferred hydrophilic polymers are those selected from the group consisting of poly(vinyl lactams) [e.g. PVP], PEO, polyoxazolines, PVOH, and polyacrylates. The currently most preferred hydrophilic polymer is PEO.

When PEO is used as hydrophilic polymer, it may be of any suitable weight average molecular weight (Mw), but preferably in the range of 100,000 to 8,000,000, most preferably 200,000 to 4,000,000 as measured by standard GPC setup. Suitable PEOs may be purchased from Dow under the trade name Polyox®.

When PVP is used as a hydrophilic polymer, it may be of any suitable weight average molecular weight (Mw), but preferably in the range of 10,000 to 3,500,000. Suitable PVPs may be purchased from ISP Corp. under the trade name Plasdone.

It should be understood that the expression "a hydrophilic polymer" and the like is intended to encompass a single hydrophilic polymer as well as a mixture of two or more hydrophilic polymers.

If the prefabricated shaped article is made of a polyolefin, polyolefins will typically be preferred as a thermoplastic substrate polymer and often in combination with more polar polymers or polymers with functional groups, which can introduce compatibility with the final hydrophilic lubricious coating.

When the term "polymer" is used herein, e.g. in connection with the expression "hydrophilic polymer", it typically implies that the weight average molecular weight is more than 10 kDa. The molecular weight limit range provided for "polymers" is hence complementary to the limit given for "low molecular weight", i.e. up to 10 kDa".

### Scaffolds having photo-initiator moieties covalently linked thereto and/or covalently incorporated therein

The coating composition further comprises - as one of the principal constituents - one or more low molecular weight scaffolds having a plurality of photo-initiator moieties covalently linked thereto and/or covalently incorporated therein.

The scaffold may be chosen from a wide range of linear, branched, cyclic and dendritic molecular species, i.e. the photo-initiator moieties are "covalently linked" to such scaffolds. It should be possible to bind a plurality of (i.e. at least two) photo-initiator moieties to the scaffold(s) by covalent bonds. Moreover, the scaffold may be in the form of two or several scaffold fragments which are held together by photo-initiator moieties, i.e. the photo-initiator moieties are "covalently incorporated" into the backbone of the scaffold. It can easily be envisaged that scaffolds may have photo-initiator moieties covalently linked thereto and at the same time may have photo-initiator moieties covalently incorporated therein.

An illustrative example of a scaffold having photo-initiator moieties covalently linked thereto is e.g.:

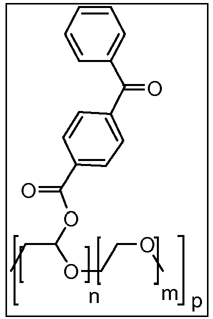

An illustrative example of a scaffold having photo-initiator moieties covalently incorporated in the backbone thereof is e.g.:

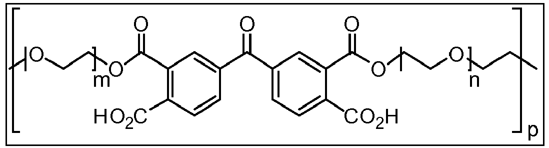

The scaffold should be capable of having covalently linked thereto and/or covalently incorporated therein a plurality of photo-initiator moieties. The "plurality" of photo-initiator moieties means at least two photo-initiator moieties, but in some instances more than two (e.g. three, four, five, six or even more) photo-initiator moieties.

In some currently preferred embodiments, the scaffold has at least three photo-initiator moieties covalently linked thereto and/or covalently incorporated therein.

With respect to the "loading" of photo-initiator moieties, the photo-initiator moieties constitute 0.01-20 % by weight, such as 0.05-15 % of the combined amount of the hydrophilic polymer(s), and the one or more low molecular weight scaffolds (including the photo-initiator moieties).

The term "low molecular weight" refers to a scaffold (without the photo-initiator moieties) having in itself a weight average molecular weight (Mw) of up to 10 kDa (10,000 g/mol). Preferably, the weight average molecular weight of the scaffold is in the range of 50-10,000 Da (g/mol), such as 100-10,000 Da (g/mol), in particular 250-8,000 Da (g/mol) or 500-10,000 Da (g/mol). It should be understood that weight average molecular weight of the "scaffold" refers to the weight of the scaffold without the photo-initiator moieties, or the total weight of the scaffold fragments without the photo-initiator moieties, whatever the case may be.

If the scaffold is in the form of two or more scaffold fragments it is furthermore preferred that each of the fragments has a molecular weight of at least 50 g/mol, such as at least 100 g/mol.

We have found that by including photo-initiator moieties, which are covalently linked to and/or covalently incorporated into a low molecular weight scaffold in the coating composition, we ensure that the photo-initiator moieties are homogeneously distributed within the coating composition. Furthermore, subsequent migration of the photo-initiator moieties is markedly reduced. Moreover, it appears that photo-initiator moieties, which for some reason remain unreacted after the irradiation, will not migrate out of the resulting coating.

In one embodiment of the present invention, the scaffold has a plurality (e.g. at least three) of photo-initiator moieties covalently linked thereto.

In another embodiment of the present invention, the scaffold has a plurality (e.g. at least three) of photo-initiator moieties covalently incorporated therein.

In a third interesting embodiment of the present invention, the scaffold has a plurality (e.g. at least three) of photo-initiator moieties, at least one being covalently linked thereto and at least one being covalently incorporated therein.

Although the scaffold may be based on a wide range of structures, including oligomers and low-molecular weight polymers (Mw < 10,000), it is currently believed that particularly useful scaffolds are those selected from polyethylene glycols, poly(styrene-co-maleic anhydride)s, aliphatic polyether urethanes, polyetheramines (e.g. Jeffamines from Huntsman), and polyesters.

The scaffold(s) may be either hydrophilic or hydrophobic or both (i.e. amphiphilic). Preferably the scaffold(s) are compatible with the polymer constituent(s) in order to ensure perfect homogeneity and hence a uniform spatial distribution of the attached photo-initiator moieties in the coating composition. If a uniform distribution of the photo-initiator moieties in the coating composition can be achieved, then the amount of photo-initiator and/or the UV irradiation time necessary for curing is minimal.

Some commercially available scaffolds with a weight average molecular weight of less than 10 kDa are listed below. These scaffolds are available from the Sigma-Aldrich Chemical Company, except where otherwise stated. Some scaffolds are listed in more than one category.

Nucleophilic scaffolds containing hydroxyl or amino groups, either as end groups or in the backbone, include: PVOH, poly(diethylene glycol/trimethylolpropane-alt-adipic acid), poly(diethylene glycol/glycerol-alt-adipic acid), PEG, [di{poly(ethylene glycol)} adipate], poly(ethylene glycol-ran-propylene glycol), poly(ethylene glycol)-block-poly(propylene glycol)-block-poly(ethylene glycol), poly(propylene glycol)-block-poly(ethylene glycol)-block-poly(propylene glycol), poly(propylene glycol), poly(tetrahydrofuran), [polycaprolactone diol], [polycaprolactone triol], [poly(diethylene glycol phthalate)diol], poly(4-hydroxystyrene), [polybutadiene, dihydroxyl terminated], [HPEU with hydroxyl end groups], [polyurethane diol solution (proprietary Aldrich product)], sugars, dextrans, pullulans, chitosan oligosaccharide lactate, gelatins (from Fibrogen), hydroxypropyl methylcellulose, [poly(tetrahydrofuran), bis(3-amino-1-propyl) terminated], [poly(ethyleneimine), ethylenediamine end-capped], [poly(propylene glycol)-block-poly(ethylene glycol), bis(3-amino-1-propyl) terminated], [poly(propylene glycol)-block-poly(ethylene glycol)-block-poly(propylene glycol), bis(2-amino-1-propyl) terminated], and [poly(propylene glycol), bis(2-amino-1-propyl) terminated]. Notably, dendritic polyols such as Boltorn H20, Boltorn H30 and Boltorn H40 (from Perstorp), and Starburst, Priostar DNT-2210 and Priostar DNT-2211 (from Dendritic Nanotechnologies) constitute very good scaffolds for the present invention. Dendritic polyamines such as the Starburst series, Priostar DNT-2200 and Priostar DNT-2201 (from Dendritic Nanotechnologies) also constitute good scaffolds for the invention. Hyperbranched polynucleophiles may also be used.

Electrophilic scaffolds containing carboxylic acids, anhydrides or isocyanate groups, either as end groups or in the backbone, include: Poly(acrylic acid), [poly(acrylic acid) sodium salt], [poly(methacrylic acid) sodium salt], [poly(styrenesulfonic acid) sodium salt], poly(acrylic acid-co-maleic acid), [poly(acrylonitrile-co-butadiene-co-acrylic acid), dicarboxy terminated], polystyrene-block-poly(acrylic acid), gelatins (from Fibrogen), [poly(ethylene glycol), di(carboxymethyl) terminated], [poly(acrylonitrile-co-butadiene), dicarboxy terminated], [polybutadiene, dicarboxy terminated], poly(isobutylene-alt-maleic anhydride), [poly(ethylene adipate), tolylene 2,4-diisocyanate terminated], and [poly(propylene glycol), tolylene 2,4-diisocyanate terminated]. Dendritic polycarboxylic acids such as the Starburst series, Priostar DNT-2220 and Priostar DNT-2221 (from Dendritic Nanotechnologies) also constitute good scaffolds for the invention. Hyperbranched polyelectrophiles may also be used.

Scaffolds suitable for transesterification and transamidation include: Poly(methyl methacrylate), poly(ethyl methacrylate), poly(butyl methacrylate), and poly(tert-butyl methacrylate).

Scaffolds containing acylatable, electron-rich aromatic systems include: Polystyrene-block-poly(acrylic acid), poly(2-vinylpyridine), poly(2-vinylcarbazole), polycarbonate, poly(α-methylstyrene), polystyrene, poly(2-vinylnaphthalene), and polyacenaphthylene.

Scaffolds containing graftable ether linkages: PEG, HPEU, [di{poly(ethylene glycol)} adipate], poly(ethylene glycol-ran-propylene glycol), poly(ethylene glycol)-block-poly(propylene glycol)-block-poly(ethylene glycol), poly(propylene glycol)-block-poly(ethylene glycol)-block-poly(propylene glycol), poly(propylene glycol), poly(tetrahydrofuran), and [poly(diethylene glycol phthalate) diol].

Further nucleophilic scaffolds with a weight average molecular weight of less than 10 kDa, may be formed by radical homopolymerization, random copolymerisation or block copolymerisation of at least one of the monomers 2-hydroxyethyl acrylate, 2-hydroxyethyl methacrylate, and 4-hydroxystyrene. If a copolymer is made, it may also contain one or more of the following monomers with relatively inert side chains: Styrene, α-methylstyrene, ring-alkylated styrenes such as vinyltoluene, vinylpyridines, vinylimidazole, (meth)acrylic esters such as methyl methacrylate, (meth)acrylic amides such as acrylamide, amides of vinylamine such as N-vinylformamide, vinyl nitriles such as acrylonitrile, vinyl esters such as vinyl acetate, ethylene, propylene, 1-butene, isobutylene, butadiene, isoprene, chloroprene, and vinyl halides such as vinyl chloride.

Further electrophilic scaffolds containing carboxylic acids, sulphonic acids or phosphonic acids, and with a weight average molecular weight of less than 10 kDa, may be formed by radical homopolymerization, random copolymerisation or block copolymerisation of at least one of the monomers (meth)acrylic acid, maleic acid, fumaric acid, crotonic acid, tiglic acid, itaconic acid, S-vinylsulphonic acid, vinylbenzenesulphonic acid, 2-acrylamido-2-methylpropanesulphonic acid (AMPS), 2-sulphoethyl methacrylate, N,N-dimethyl-N-methacryloyloxyethyl-N-(3-sulphopropyl)ammonium betaine (SPE), and P-vinylphosphonic acid. If a copolymer is made, it may also contain one or more of the monomers with relatively inert side chains that were mentioned under "Further nucleophilic scaffolds" above.

Further scaffolds suitable for transesterification and transamidation, and with a weight average molecular weight less than 10 kDa, may be formed by radical homopolymerization, random copolymerisation or block copolymerisation of at least one monomer belonging to the groups of alkyl (meth)acrylates, alkyl crotonates, alkyl tiglates, dialkyl maleate, dialkyl fumarate, and dialkyl itaconate. If a copolymer is made, it may also contain one or more of the following monomers , whose side chains should not affect transesterification or transamidation: Styrene, α-methylstyrene, ring-alkylated styrenes such as vinyltoluene, vinylpyridines, vinylimidazole, (meth)acrylic esters such as methyl methacrylate, (meth)acrylic amides such as acrylamide, amides of vinylamine such as N-vinylformamide, vinyl nitriles such as acrylonitrile, ethylene, propylene, 1-butene, isobutylene, butadiene, isoprene, chloroprene, and vinyl halides such as vinyl chloride.

Further scaffolds containing acylatable, electron-rich aromatic systems with a weight average molecular weight of less than 10 kDa may be formed by radical homopolymerization, random copolymerisation or block copolymerisation of at least one styrene monomer, such as styrene, α-methylstyrene, ring-alkylated styrenes, or 4-hydroxystyrene. If a copolymer is made, it may also contain one or more of the following monomers with non-acylatable side chains: (Meth)acrylic esters such as methyl methacrylate, (meth)acrylic amides such as acrylamide, amides of vinylamine such as N-vinylformamide, vinyl nitriles such as acrylonitrile, vinyl esters such as vinyl acetate, ethylene, propylene, 1-butene, isobutylene, butadiene, isoprene, chloroprene, and vinyl halides such as vinyl chloride.

Further scaffolds containing graftable ether linkages with a weight average molecular weight of less than 10 kDa may be formed by radical homopolymerization, random copolymerisation or block copolymerisation of at least one of the monomers PEG methacrylate, PEG methyl ether methacrylate, PEG ethyl ether methacrylate, PEG methyl ether acrylate, PEG phenyl ether acrylate, poly(propylene glycol) methacrylate, poly(propylene glycol) acrylate, and poly(propylene glycol) methyl ether acrylate. If a copolymer is made, it may also contain one or more of the non-graftable monomers styrene, α-methylstyrene, ring-alkylated styrenes such as vinyltoluene, vinylpyridines, vinylimidazole, (meth)acrylic amides such as acrylamide, amides of vinylamine such as N-vinylformamide, vinyl nitriles such as acrylonitrile, ethylene, propylene, 1-butene, isobutylene, butadiene, isoprene, chloroprene, and vinyl halides such as vinyl chloride, (meth)acrylic acid, maleic acid, fumaric acid, crotonic acid, tiglic acid, itaconic acid, S-vinylsulphonic acid, vinylbenzenesulphonic acid, 2-acrylamido-2-methylpropanesulphonic acid (AMPS), 2-sulphoethyl methacrylate, N,N-dimethyl-N-methacryloyloxyethyl-N-(3-sulphopropyl)ammonium betaine (SPE), and P-vinylphosphonic acid.

### Photo-initiators

The main function of the photo-initiator moieties is to ensure good cross-linking of the thermoplastic, hydrophilic coating to itself and to the substrate in order to obtain good cohesion and good adhesion to the substrate. The preferred properties of the photo-initiator(s) are: (i) good overlap between the lamp emission spectrum and the photo-initiator absorption spectrum; (ii) small overlap or no overlap between the photo-initiator absorption spectrum and the intrinsic, combined absorption spectrum of the other components of the coating (i.e. poly(ethylene oxide)); and good compatibility of the photo-initiator moieties including the scaffold to which the moieties are covalently linked with the poly(ethylene oxide)(s) of the coating.

The photo-initiators should efficiently transform light from the UV or visible light source to reactive radicals which can abstract hydrogen atoms and other labile atoms from polymers and hence effect covalent cross-linking. Optionally, amines, thiols and other electron donors may be added. Radical photo-initiators can be classified as either cleavable (Norrish type I reaction) or non-cleavable (of which the Norrish type II reaction is a special case, see e.g. A. Gilbert, J. Baggott: "Essentials of Molecular Photochemistry", Blackwell, London, 1991). Upon excitation, cleavable photo-initiators spontaneously break down to two radicals, at least one of which is reactive enough to abstract a hydrogen atom from most substrates. Benzoin ethers (including benzil dialkyl ketals), phenyl hydroxyalkyl ketones and phenyl aminoalkyl ketones are important examples of cleavable photo-initiators. Addition of electron donors is not required but may enhance the overall efficiency of cleavable photo-initiators according to a mechanism similar to that described for the non-cleavable photo-initiators below.

Recently a new class of β-keto ester based photo-initiators has been introduced by M.L Gould, S. Narayan-Sarathy, T.E. Hammond, and R.B. Fechter from Ashland Specialty Chemical, USA (2005): "Novel Self-Initiating UV-Curable Resins: Generation Three", Proceedings from RadTech Europe 05, Barcelona, Spain, October 18-20 2005, vol. 1, p. 245-51, Vincentz. After base-catalyzed Michael addition of the ester to polyfunctional acrylates a network is formed with a number of quaternary carbon atoms, each with two neighbouring carbonyl groups. Upon UV or visible light excitation these photo-initiators cleave predominantly by a Norrish type I mechanism and cross-link further without any conventional photo-initiator present, and thick layers may be cured. Such self-initiating systems are within the scope of the present invention.

Excited non-cleavable photo-initiators do not break down to radicals but abstract a hydrogen atom from an organic molecule or, more efficiently, abstract an electron from an electron donor (such as an amine or a thiol). The electron transfer produces a radical anion on the photo-initiator and a radical cation on the electron donor. This is followed by proton transfer from the radical cation to the radical anion to produce two uncharged radicals; of these the radical on the electron donor is sufficiently reactive to abstract a hydrogen atom from most substrates. Benzophenones, thioxanthones, xanthones, anthraquinones, fluorenones, dibenzosuberones, benzils, and phenyl ketocoumarins are important examples of non-cleavable photo-initiators. Most amines with a C-H bond in α-position to the nitrogen atom and many thiols will work as electron donors.

Another self-initiating system based on maleimides has also been identified by C.K. Nguyen, W. Kuang, and C.A. Brady from Albemarle Corporation and Brady Associates LLC, both USA (2003): "Maleimide Reactive Oligomers", Proceedings from RadTech Europe 03, Berlin, Germany, November 3-5, 2003, vol. 1, p. 589-94, Vincentz. Maleimides initiate radical polymerization mainly by acting as non-cleavable photo-initiators and at the same time spontaneously polymerize across the maleimide double bond by radical addition. In addition, the strong UV absorption of the maleimide disappears in the polymer, i.e. maleimide is a photobleaching photo-initiator; this could make it possible to cure thick layers. Such maleimide-containing systems are within the scope of the present invention.

A blend of several photo-initiators may exhibit synergistic properties, as is e.g. described by J.P. Fouassier: "Excited-State Reactivity in Radical Polymerisation Photo-initiators", Ch. 1, pp. 1-61, in "Radiation curing in Polymer Science and technology", Vol. II ("Photo-initiating Systems"), ed. by J.P. Fouassier and J.F. Rabek, Elsevier, London, 1993. Briefly, efficient energy transfer or electron transfer takes place from one photo-initiator to the other in the pairs [4,4'-bis(dimethyl¬amino)benzophenone + benzophenone], [benzophenone + 2,4,6-tri¬methyl¬benzophenone], [thioxanthone + methylthiophenyl morpholinoalkyl ketone]. However, many other beneficial combinations may be envisaged.

Furthermore, it has recently been found that covalently linked Irgacure 2959 and benzophenone in the molecule 4-(4-benzoylphenoxyethoxy)phenyl 2-hydroxy-2-propyl ketone gives considerably higher initiation efficiency of radical polymerization than a simple mixture of the two separate compounds, see S. Kopeinig and R. Liska from Vienna University of Technology, Austria (2005): "Further Covalently Bonded Photoinitiators", Proceedings from RadTech Europe 05, Barcelona, Spain, October 18-20 2005, vol. 2, p. 375-81, Vincentz. This shows that different photo-initiators may show significant synergistic effects when they are present in the same oligomer or polymer. Such covalently linked photo-initiators are also applicable within the present invention.

Hence, in one interesting embodiment of the invention, the photo-initiator moieties include at least two different types of photo-initiator moieties. Preferably the absorbance peaks of the different photo-initiators are at different wavelengths so the total amount of light absorbed by the system increases. The different photo-initiators may be all cleavable, all non-cleavable, or a mixture of cleavable and non-cleavable.

The preferred cleavable photo-initiators are benzoin ethers (including benzil dialkyl ketals) such as Irgacure 651 (Ciba); phenyl hydroxyalkyl ketones such as Darocur 1173, Irgacure 127, Irgacure 184, and Irgacure 2959 (all from Ciba), and Esacure KIP 150 and Esacure One (both from Lamberti); phenyl aminoalkyl ketones such as Irgacure 369 (Ciba), Irgacure 379 (Ciba), and Chivacure 3690 (from Double Bond Chemical); methylthiophenyl morpholinoalkyl ketones such as Irgacure 907 (Ciba) and Chivacure 3482 (Double bond Chemicals); and mono- or dibenzoylphosphinoxides such as Irgacure 819 and Darocur TPO (both from Ciba).

The preferred non-cleavable photo-initiators are benzophenone, 4-benzoylbenzoic acid (= 4-carboxybenzophenone) and esters thereof, 2-benzoylbenzoic acid (= 2-carboxybenzophenone) and esters thereof, 4,4'-bis(dimethyl-amino)benzo-phenone (Michler's ketone), 2,4,6-tri¬methyl-benzophenone, BTDA, Omnipol BP (IGM Resins), and other benzophenone derivatives; thioxanthones such as Omnipol TX (IGM Resins) and 2-carboxymethoxythioxanthone (Pentagon Fine Chemical); xanthones; anthraquinones; fluorenones; dibenzosuberones; benzils and other α-diketo compounds such as camphorquinone; and phenyl ketocoumarins. The preferred optional electron donors are benzocaine (ethyl 4-aminobenzoate), PVP-DMAEMA, tribenzylamine, triethanolamine, 2-(N,N-dimethylamino)ethanol, and N,N-dimethylethylenediamine.

The currently most preferred photo-initiators are those selected from the group Irgacure 2959, BTDA and derivatives thereof, 4-carboxybenzophenone and derivatives thereof, 2-carboxybenzophenone and derivatives thereof, and 2-carboxymethoxythioxanthone and derivatives thereof.

### Modification of photo-initiators to be suitable for covalent bonding to a scaffold

Most common photo-initiators, such as benzoin ethers (e.g. Irgacure 651, cleavable), hydroxyalkyl phenyl ketones (e.g. Darocur 1173, cleavable), benzophenones (e.g. benzophenone, non-cleavable), and thioxanthones (e.g. 2-isopropylthioxanthone, non-cleavable), have no functional groups and therefore cannot be easily bonded to the scaffold. For this reason photo-initiators with one or more functional groups are preferred. The number of commercially available photo-initiators with functional groups is limited, perhaps because photo-initiators have traditionally been employed as monofunctional, non-polymerized ingredients in coating compositions. Hence it may be necessary to custom synthesize certain functional photo-initiators in order to be able to bind them to the scaffold.

Whereas a vast number of chemical reactions, which form covalent bonds between two separate compounds, are known, the present invention focuses on the presence of either a primary hydroxyl or amino group (i.e. a strong nucleophile) or a reactive carboxylic acid derivative, such as an anhydride or an acid chloride (i.e a strong electrophile), in the photo-initiator. The following examples will illustrate this:

Irgacure 2959 (from Ciba) is a Norrish type I photo-initiator which contains a nucleophilic primary hydroxyl group:

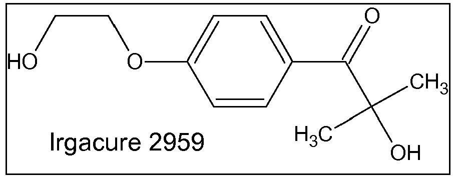

If stronger nucleophilicity is needed, Irgacure 2959 may be sulfonated and then transformed into the corresponding primary amine, e.g. by the Gabriel synthesis (see e.g. J. March: "Advanced Organic Chemistry. Reaction, Mechanisms, and Structure", 3. ed., p. 377-9, Wiley-Interscience, New York, 1985):

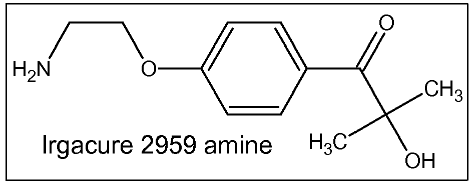

The hydroxyl group in Irgacure 2959 may be functionalized to an electrophilic acid derivative in several ways, so that it may react with free hydroxyl and amino groups:
1. The acid derived from Cr(VI)-oxidation of Irgacure 2959: 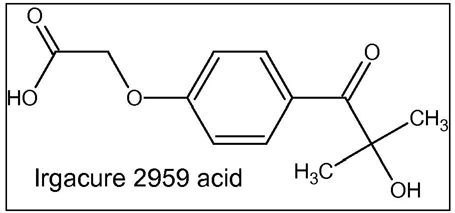
2. The acid derived from 1:1 reaction between Irgacure 2959 and succinic anhydride: 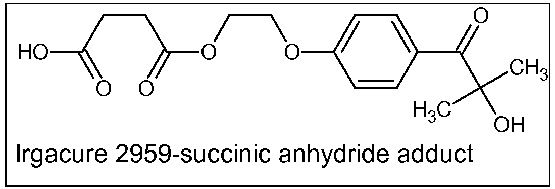
3. The acid derived from 1:1 reaction between Irgacure 2959 and maleic anhydride:

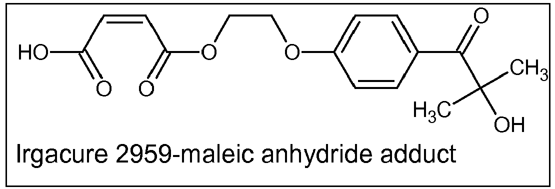

These acids may conveniently be turned into the corresponding reactive acid chlorides by treatment with SOCl₂. Care must be taken to use the acid chlorides soon after formation to avoid reaction between the acid chloride part and the tertiary hydroxyl group in the hydroxyalkyl part of the ketone.

Conversely, electrophilic 2-, 3- or 4-benzoylbenzoyl chloride (formed by reaction between SOCl₂ and commercially available 2-, 3- or 4-benzoylbenzoic acid, which are derivatives of the non-cleavable photo-initiator benzophenone), may be transformed into a nucleophile by slow addition to a large excess of ethylene glycol in order to form the corresponding 2-hydroxyethyl benzoylbenzoates, e.g.:

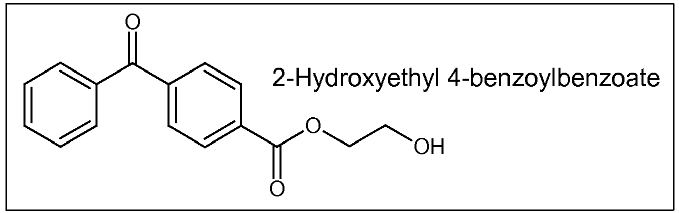

If ethanolamine or ethylenediamine is used instead of ethylene glycol, then the corresponding N-(2-hydroxyethyl)benzoylbenzamides and N-(2-aminoethyl)benzoyl-benzamides may be formed. All these nucleophilic derivatives may e.g. react with polyanhydrides such as poly(styrene-co-maleic anhydride) (SMA) (see further below), and with isocyanates. Alternatively, 2-, 3- or 4-hydroxybenzophenone or 2-, 3- or 4-amino-benzophenone may be obtained commercially and used directly, although the nucleophilicity of these hydroxyl and amino groups will be considerably smaller than that of the ethylene glycol, ethanolamine and ethylenediamine derivatives mentioned above.

Thioxanthones are also very interesting, non-cleavable photo-initiators because they absorb near 400 nm and hence may be cured by UV-A light or by visible, blue light. An example of a derivative of thioxanthone is 2-carboxymethoxyxanthone, which may be transformed into the electrophilic acid chloride and further, if desired, into nucleophilic species by reaction with excess ethylene glycol (to form 2-hydroxyethyl thioxanthon-2-yloxyacetate), ethanolamine (to form N-(2-hydroxyethyl)thioxanthon-2-yloxyacetamide), or ethylenediamine (to form N-(2-aminoethyl)thioxanthon-2-yloxyacetamide), as described above.

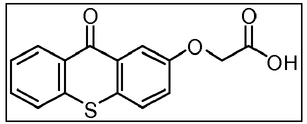

### Examples of coupling between photo-initiator moieties and scaffolds

Nucleophilic scaffolds, such as Boltorn H20 with 16 free OH groups, may react directly with electrophilic photo-initiators, such as 4-benzoylbenzoyl chloride, to form a photoactive polyester:

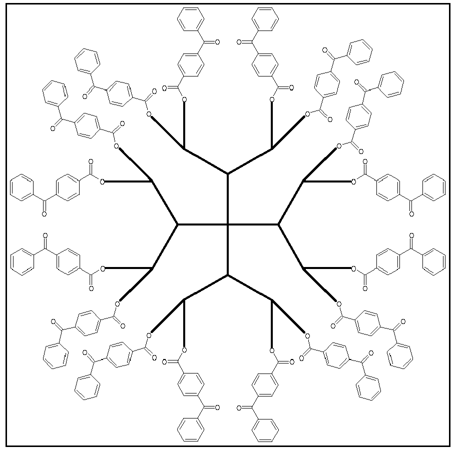

The degree of photo-initiator substitution on the polyol can be controlled if the acid chloride is added to the Boltorn solution.

The acidic components of the electrophilic scaffolds, such as the carboxylic acid groups in poly(acrylic acid), may be transformed to the corresponding acid chlorides, sulphonyl chlorides or phosphonyl chlorides by treatment with SOCl₂ or PCl₅. Alternatively, the acids may be treated with a dehydrating agent, such as N,N'-dicyclohexylcarbodiimide, to form species resembling acid anhydrides in reactivity towards nucleophiles. Such acid chlorides, sulphonyl chlorides and phosphonyl chlorides and the corresponding anhydrides are activated towards reaction with nucleophilic photo-initiators, such as Irgacure 2959, to form the respective esters, amides, sulphonyl esters, sulphonamides, phosphonyl esters, and phosphonamides:

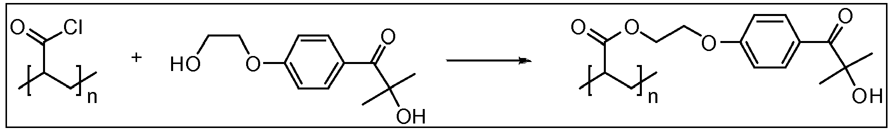

Photoactive esters and amides may be formed with excess photo-active nucleophiles by transesterification or transamidation of esters from the scaffold. Catalysts (such as manganese or zinc salts) may be added and a vacuum may be applied if the photo-inactive component to be removed has a lower boiling point than the photoactive component so as to remove the photo-inactive component from the equilibrium. The two reactions may be represented as follows:

Scaffold-CO-OR + HO-Photo-initiator → Scaffold-CO-O-Photo-initiator + HO-R (transesterification)

Scaffold-CO-OR + H₂N-Photo-initiator → Scaffold-CO-NH-Photo-initiator + HO-R (transamidation)

"Scaffold-CO-OR" may be e.g. poly(diethyl maleate) with a weight average molecular weight not exceeding 10 kDa. "HO-Photo-initiator" may be e.g. Irgacure 2959, 2- or 4-hydroxybenzophenone, 2-hydroxyethyl 4-benzoylbenzamide, N-(2-hydroxyethyl)-2-benzoylbenzamide, 2-hydroxyethyl thioxanthon-2-yloxyacetate, or N-(2-hydroxyethyl)thioxanthon-2-yloxyacetamide. "H₂N-Photo-initiator" may be e.g. Irgacure 2959 amine, N-(2-aminoethyl)-4-benzoylbenzamide, or N-(2-aminoethyl)thioxanthon-2-yloxyacetamide:

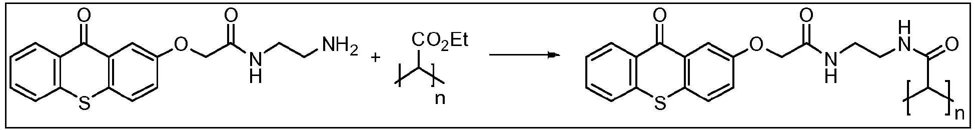

Ethers such as PEG or poly(propylene glycol) may be acyloxylated by reaction with a tert-butyl peroxyester of a carboxyl-containing photo-initiator to give the ether ester and tert-butyl alcohol (see J. March: "Advanced Organic Chemistry. Reaction, Mechanisms, and Structure", 3. ed., p. 636-7, Wiley-Interscience, New York, 1985). As an example, the coupling with a benzophenone derivative (2-benzoylbenzoyl chloride) is shown here:

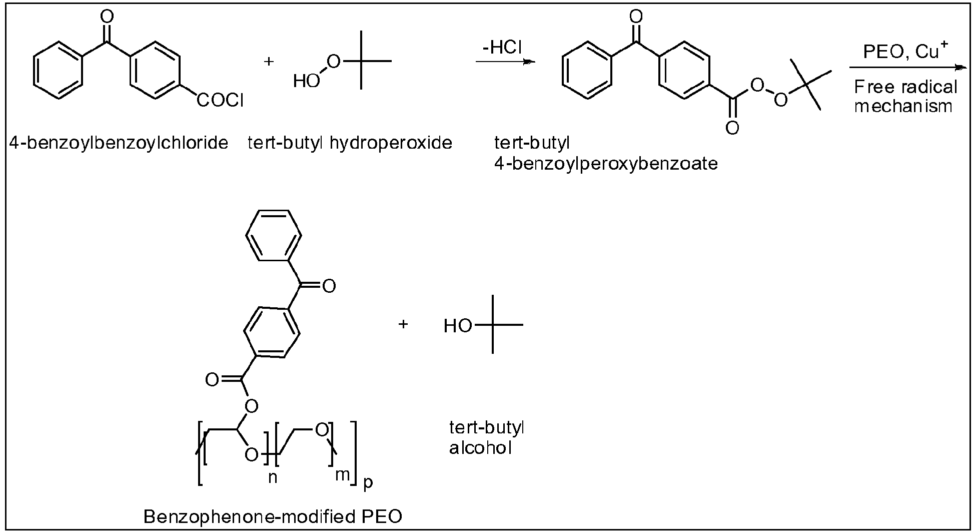

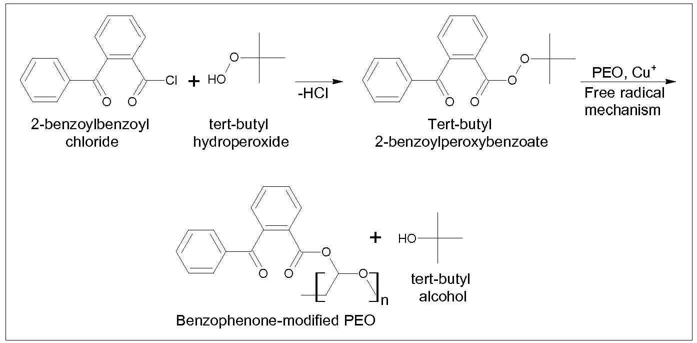

The reaction may also be carried out with BTDA or with an acid chloride derivative of a Norrish type I photo-initiator, such as Irgacure 2959 acid chloride.

Ethers such as PEG or poly(propylene glycol) may alkylate (i.e. add to) photo-initiator double bonds in the presence of peroxides to provide the corresponding alkylated ethers. The best results are obtained with electron-deficient alkenes such as maleic anhydride (see C. Walling, E.S. Huyser (1963): "Free radical additions to olefins to form carbon-carbon bonds", Organic Reactions, 13, 91-149). A nucleophilic photo-initiator (such as Irgacure 2959) may e.g. acquire an electron-deficient double bond by esterification with maleic anhydride.

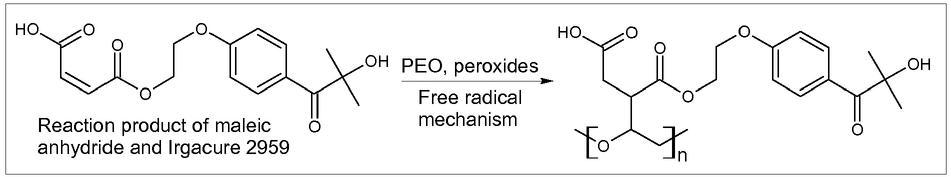

Isocyanate-capped, low-molecular HPEU as the scaffold may also be functionalized with a nucleophilic photo-initiator (such as Irgacure 2959) at both ends to form a photo-active polyurethane:

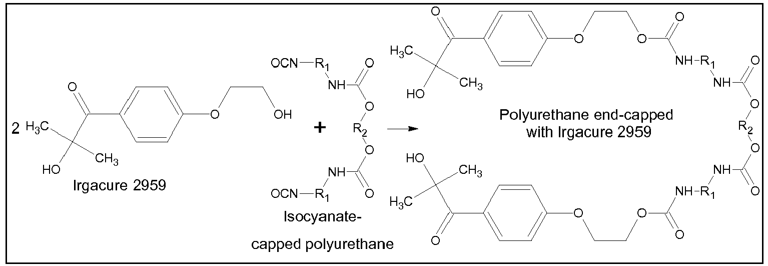

Similarly, the side chains of the scaffold poly(styrene-co-maleic anhydride) (SMA) may be modified with a nucleophilic photo-initiator (such as Irgacure 2959 or modified benzophenones):

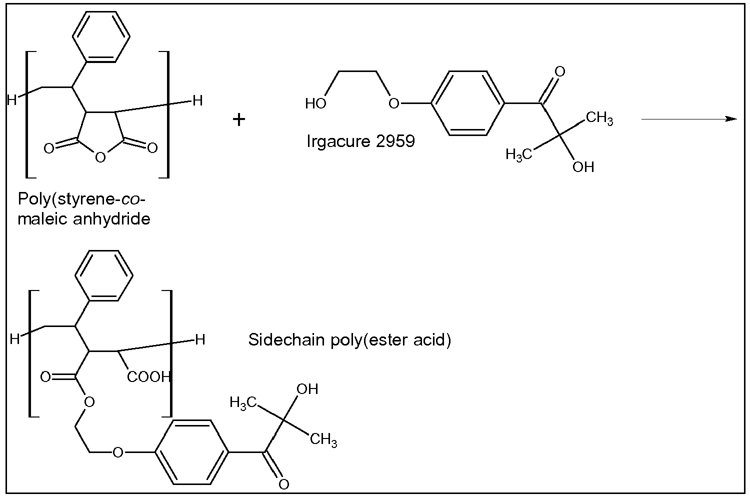

### Examples of transformation of a scaffold to a photo-initiator

Benzophenones may be formed in situ by Friedel-Crafts benzoylation of an electron-rich aromatic moiety with benzoyl chloride and a Lewis acid as catalyst, e.g. AlCl₃. Aromatic anhydrides, such as phthalic anhydride, pyromellitic dianhydride (1,2,4,5-benzenetetra-carboxylic acid dianhydride) and BTDA, are less reactive than benzoyl chloride but may also be used. If the para position of the aromatic moiety is vacant, then the para compound is the main product because of the size of the benzoyl group (see e.g. J. March: "Advanced Organic Chemistry. Reaction, Mechanisms, and Structure", 3. ed., p. 484-7, Wiley-Interscience, New York, 1985). However, the method may also be used with aromatic moieties which do not have a vacant para position. The aromatic moiety may be part of homo- or copolymers of vinylpyridine, styrene, α-methylstyrene, vinyltoluene, alkoxystyrene, aryloxystyrene, ethylstyrene, tert-butylstyrene, isopropylstyrene, dimethylstyrene, and other alkylated styrenes. Any aromatic diisocyanates or aromatic diols, that have been employed in the production of HPEU, may also be benzoylated. The aromatic ring of the benzoyl chloride may also itself be substituted; electron donating substituents on the benzoyl chloride will increase the rate of reaction. As an example, the following reaction occurs with ordinary polystyrene:

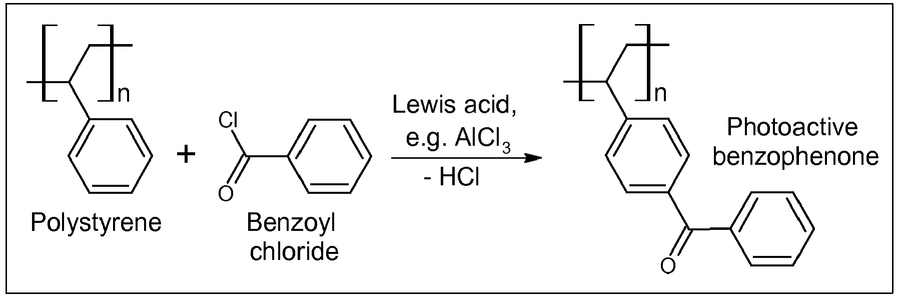

Correspondingly, α,α-dialkyl-α-hydroxy substituted acetophenones (i.e. cleavable photo-initiators) may also be formed in situ by Friedel-Crafts acylation of an electron-rich aromatic moiety with the relevant α,α-dialkyl-α-hydroxyacetylchloride. For example, in order to make a 2-hydroxy-2-propyl phenyl ketone, the electron-rich aromatic moiety must be treated with 2-hydroxy-2-methylpropionyl chloride (=2-hydroxyisobutyryl chloride = α-hydroxyisobutyryl chloride). The precursor of this acid chloride, α-hydroxyisobutyric acid, is e.g. available from Sigma-Aldrich.

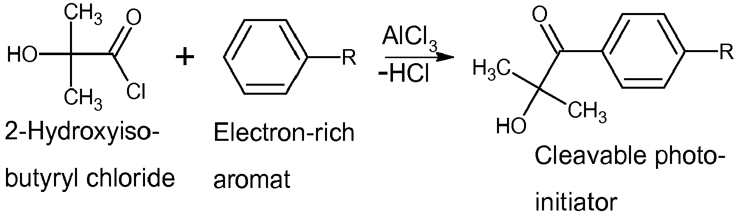

Care must be taken that the acid chloride, once formed, does not react with the tertiary hydroxyl group to form the polyester poly(2-isobutyrate).

### Examples of synthesis of a scaffold with photo-initiator incorporated in the backbone

A difunctional, electrophilic photo-initiator, such as BTDA, may react with a dihydroxy- or diamino-terminated, nucleophilic scaffold fragment, e.g. a low-molecular HPEU, to form the corresponding photo-initiator-containing scaffold:

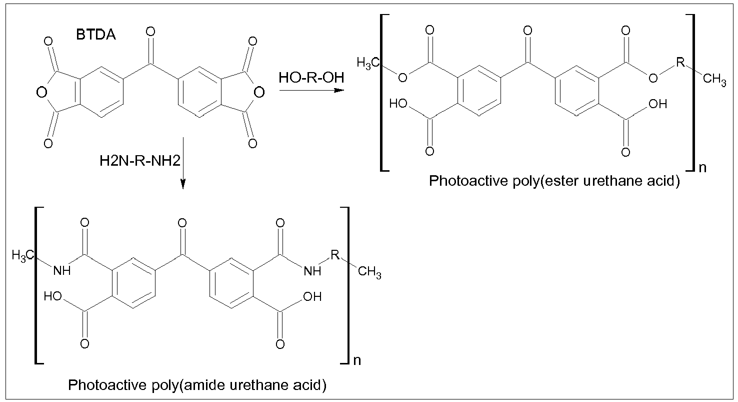

The resulting scaffolds have photo-initiating moieties in the backbone instead of in the side chains. Such scaffolds are within the scope of the present invention. The reactions run best in polar organic solvents such as DMSO, DMA, DMF, NMP and pyridine.

The cross-linking reaction of the photoactive BTDA-based poly(ester urethane acid) will be:

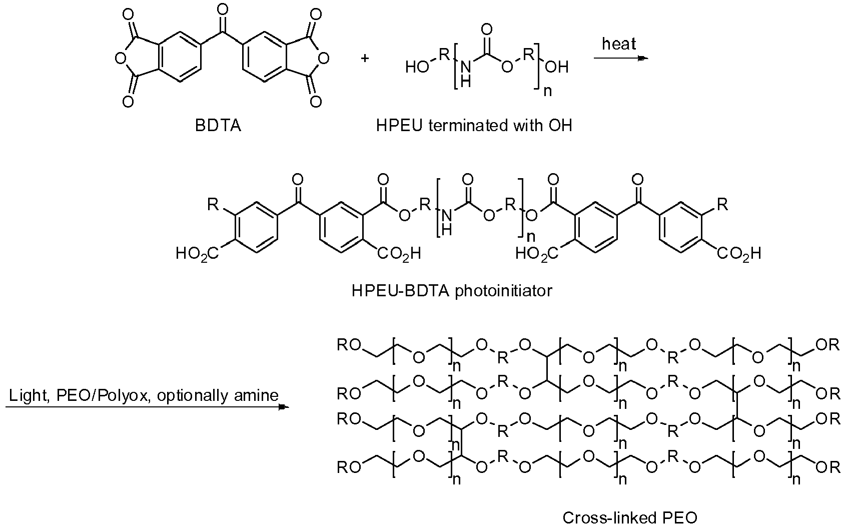

Jeffamine D-230 (from Huntsman; hydrophobic; a = 2-3, b = c = 0), which is shown below, also reacts willingly with BTDA:

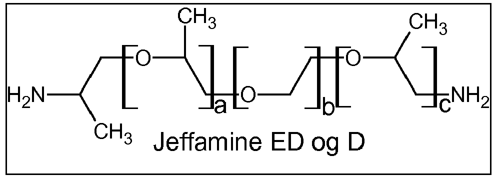

As mentioned above, BTDA may also react with the hydroxyl end groups of low molecular weight scaffold fragments, such as low-molecular PEG and other low molecular weight polyethers. Upon photo-curing of PEO with a BTDA-containing scaffold, a stable, cross-linked, hydrophilic polymer network is formed, which becomes very slippery when wet.

### Detailed procedure for the preparation of a medical device element

### Step (i)

In an initial step of the method, the prefabricated shaped article and/or the thermoplastic substrate polymer are provided.

As it is clear from the section "Thermoplastic substrate polymer", the substrate polymer is typically a commercial product traded in a suitable physical form, e.g. as pellets, chips, granules, etc. Hence, pre-treatment or preparation is normally not necessary.

If a mixture of two or more substrate polymers is used, it is typically desirable to homogenize the polymers either in a melted form or by dissolving the polymers in a common solvent followed by solvent removal by conventional procedures and involving conventional equipment such as spray coating, roller drying or precipitation in a non-solvent. Preferably, the solvent solution is cast into a film and the solvent removed from the film by any conventional technique. Reduced pressure and/or elevated temperature may be used to aid solvent removal. The resulting homogeneous blend may be chipped or pelletized prior to melt processing.

It is further clear from the section "Prefabricated shaped article" that the shaped article is often available from commercial sources or is readily prepared as will be known by the skilled person within the relevant art. Alternatively, but also very interestingly, the shaped article may be prepared immediately prior to its use in the method of the invention, in certain embodiments even in the same process line as the one where the method is applied. Moreover, the prefabricated shaped article may be pre-treated and even pre-coated prior to use in the method of the invention.

### Step (ii)

The coating composition for the preparation of the medical device element may preferentially be prepared by standard thermoplastic compounding processes such as batch kneaders (e.g. Brabender mixers), continuous kneaders (Buss co-kneader), twin screw extruders and single screw extruders. The resulting homogeneous blend may be chipped or pelletized prior to melt processing or powder coating. It can also be delivered as a melt directly into the coating process in step (iii), e.g. a coating co-extrusion process.

Another preferred method is to supply the coating composition made by powder mixing directly into the coating process. As an option, this powder mix can be slightly sintered together by mixing it in a high speed mixer, where the heating is created by the friction, or in a low speed mixer, tumbler or fluid bed where the heat is supplied from outside, e.g. by the supplied air. These methods give the advantage of getting a more homogeneous mixing of the components and the advantage of practically eliminating the risk for blocking or settling of the components in the supply system for the coating process in step (iii), e.g. a coating co-extrusion process or e.g. a powder coating process.

The coating composition for the preparation of the medical device element may also be prepared by dissolving the constituents thereof in a common solvent. The solvent may then be removed to leave a homogeneous blend of the hydrophilic polymer, the scaffold, the one or more photo-initiators, and any additives, which is ready for extrusion. Any conventional procedure or equipment may be used for solvent removal such as spray coating, roller drying or precipitation in a non-solvent such as acetone or carbon tetrachloride. Preferably the solvent solution is cast into a film and the solvent removed from the film by any conventional technique. The cast film may then be heated in a convection oven at a temperature from ambient to about 70°C. Reduced pressure may be used to aid solvent removal. The resulting homogeneous blend may be chipped or pelletized prior to melt processing or powder coating.

This pelletized coating composition may subsequently be extruded, injection moulded or powder coated on the prefabricated shaped article or the thermoplastic substrate polymer as described for step (iii) below.

### Step (iii)

This step involves extruding, injection moulding or powder coating the thermoplastic coating composition of step (ii) on the prefabricated shaped article or together with the thermoplastic substrate polymer of step (i) so as to provide the medical device element of said prefabricated shaped article and/or substrate polymer having thereon a layer of said coating composition, wherein, when both of said prefabricated shaped article and substrate polymer are present, said prefabricated shaped article has thereon a layer of said substrate polymer.

Three main embodiments are encompassed by this step.

In a first main embodiment, only a prefabricated shaped article is provided in step (i) and step (iii) involves extruding, injection moulding or powder coating the coating composition of step (ii) on the prefabricated shaped article of step (i) so as to provide the medical device element of said prefabricated shaped article having thereon a layer of said coating composition.

In a second main embodiment, only a thermoplastic substrate polymer is provided in step (i), and step (iii) involves extruding or injection moulding the coating composition of step (ii) together with the thermoplastic substrate polymer of step (i) so as to provide the medical device element of said thermoplastic substrate polymer having thereon a layer of said coating composition.

In a third main embodiment, a prefabricated shaped article as well as a thermoplastic substrate polymer are provided in step (i), wherein step (iii) involves extruding or injection moulding the coating composition of step (ii) on the prefabricated shaped article together with the thermoplastic substrate polymer of step (i) so as to provide the medical device element of said prefabricated shaped article and said thermoplastic substrate polymer, said prefabricated shaped article having thereon a layer of said thermoplastic substrate polymer and said thermoplastic substrate polymer having thereon a layer of said coating composition.

According to one embodiment of the invention, the thermoplastic substrate is consisting of more than one layer, preferably two layers, of a thermoplastic substrate polymer. The layers may be of different or identical types of polymers.

One layer of a thermoplastic substrate polymer may be used as a tie-layer.

The three main embodiments will be discussed in the following.

In a first variant of the first main embodiment, a melt of the coating composition is extruded onto a surface of a prefabricated shaped article.

In a second variant of the first main embodiment, a melt of the coating composition is injection moulded onto a surface of a prefabricated shaped article.

In a third variant of the first main embodiment, the coating composition is powder coated onto a surface of a prefabricated shaped article.

In one variant of the second main embodiment, a melt of the thermoplastic substrate polymer and a melt of the coating composition are extruded to provide a shaped article having a coating of the coating composition on the surface of the substrate polymer.

In another variant of the second main embodiment, a melt of the thermoplastic substrate polymer and a melt of the coating composition are injection moulded to provide a shaped article having a coating of the coating composition on the surface of the substrate polymer. This interesting variant can be accomplished in a two step injection moulding process wherein the outer layer of the coating composition is first moulded followed by the moulding of the thermoplastic substrate polymer.

In one variant of the third main embodiment, a melt of the substrate polymer and a melt of the coating composition are extruded onto a surface of a prefabricated shaped article.

In another variant of the third main embodiment, a melt of the substrate polymer and a melt of the coating composition are injection moulded onto a surface of a prefabricated shaped article. This interesting variant can be accomplished in a two step injection moulding process wherein the outer layer of the coating composition is first moulded using a solid core followed by the moulding of the thermoplastic substrate polymer using the prefabricated shaped article as the core.

The coating composition may be extruded/co-extruded with the substrate polymer using any conventional and commercially available extrusion equipment.

Alternatively, the composition may be crosshead-extruded or co-extruded onto a prefabricated shaped article, e.g. polymeric article. Extrusion of a skin layer is a conventional process in which a melt of a thermoplastic material (here the thermoplastic substrate polymer or the coating composition) is metered through a die directly onto a solid, continuous, shaped surface.

Moreover, (co)extrusion and injection moulding may be conducted as described in US 5,061,424 and 6,447,835.

The coating composition may also be injection moulded so as to provide a coating on a thermoplastic substrate polymer or prefabricated shaped article. The injection moulding variants may have one or two process steps. In one variant corresponding to the second variant of the first main embodiment (see above), the coating composition is injected at high pressure into a mould, which is the inverse of the shape of the final product, using a solid core of the prefabricated shaped article. In a second variant (corresponding to the second variant of the second main embodiment (see above)), step (iii) can be accomplished in two sub-steps, namely by first moulding the coating composition using a solid core, removing the solid core, and subsequently moulding the thermoplastic substrate polymer, optionally using a slightly smaller solid core. In a third variant (corresponding to the second variant of the third main embodiment (see above)), step (iii) can be accomplished in two sub-steps, namely by first moulding the coating composition using a solid core, removing the solid core, and subsequently moulding the thermoplastic substrate polymer using the prefabricated solid article as the solid core. In a fourth variant (corresponding to the second variant of the second main embodiment (see above), step (iii) can be accomplished in two sub-steps, namely by first moulding the thermoplastic substrate polymer using a cavity of one size, removing the cavity, and subsequently moulding the coating composition onto the thermoplastic substrate polymer using a slightly larger cavity. In a fifth variant (corresponding to the second variant of the third main embodiment (see above), step (iii) can be accomplished in two sub-steps, namely by first moulding the thermoplastic substrate polymer using a cavity of one size and the prefabricated shaped article as the core, removing the cavity, and subsequently moulding the coating composition onto the thermoplastic substrate polymer using a slightly larger cavity.

With regard to powder coating, which generally follows conventional principles, the pelletized compound containing hydrophilic polymer(s) and scaffold(s) having photo-initiator moieties can be milled to a particle size in the range of 5 to 250 micrometers. Usually a powder coating composition with a particle size distribution in the range of 10 to 100 micrometers is preferred.

The powder coating compositions are typically applied by spraying or by the use of a fluidized bed system. In case of a metal substrate (prefabricated shaped article), application of the coating by electrostatic spraying is preferred. In case of spraying, the powder coating can be applied in a single sweep or in several passes to provide a film having the preferred thickness.

After applying the powder by spraying or by using a fluidized bed system or any other powder coating application technology known in the industry, the thermoplastic powder is heated to about 80 to 200 °C depending on the type of substrate in order to form a uniform coating layer about 5 to 250 micrometers thick, usually about 10 to 100 micrometers thick.

The thickness of the dry layer of the coating composition is typically 2.5-500 µm, preferably 2.5-125 µm.

The thickness of the substrate polymer (if present) is typically 5-1000 µm, more typically 10-50 µm or 100-500 µm.

The medical device element obtained by the method is dry and in general non-sticky until humidified by finger-touch or wetted with a liquid at which time it develops a slippery, lubricious surface.

The method of the invention is particularly useful for the preparation of medical device elements having the shape of a rod or tubing. For example, a catheter thus prepared becomes instantly lubricious when it comes into contact with a water-containing fluid and thereby contributes greatly to the comfort of a patient undergoing catheterization. An extruded rod in the form of a guide-wire becomes lubricious when wet and thus slides easily.

After extrusion or injection moulding it may be necessary to cool the medical device element, e.g. by cold air or in a water bath.

This being said, the currently most preferred embodiments of the step (iii) are those involving (co)extrusion.

### Step (iv)

In a subsequent step, the coating composition is irradiated with UV or visible light so as to covalently cross-link the coating composition. UV or visible light is defined as light having a wavelength of 100-750 nm. Particularly relevant wavelength ranges are 100-250 nm and 250-400 nm (both UV light), and 400-750 nm (visible light). In the present context, the terms "photo-curing", "photo-cure" and the like refer to curing by means of UV or visible light. Curing by means of UV light is preferred, although curing by means of blue light (visible light wavelength range) is equally applicable.

The UV or visible light may be applied by means of a polychromatic or monochromatic UV or visible light source, preferably with high intensity and with an emission spectrum that matches the absorbance spectrum of the photo-initiator(s) as well as possible. In the absence of reactive monomers, the cross-linking of the coating takes place only by the bimolecular combination of radicals derived from the UV (or visible light) irradiated photo-initiators. Hence, if the light intensity is doubled, the concentration of radicals is also doubled, but the amount of cross-linking reactions is quadrupled. This is why a high light intensity is preferred. Suitable polychromatic light sources include: (i) deuterium lamps, (ii) mercury lamps, possibly doped with iron, gallium or other elements that significantly affects the output spectrum, (iii) xenon arc lamps, both pulsed and unpulsed, and (iv) halogen lamps (which emit mainly visible light). Suitable monochromatic light sources include: (v) gas and solid state lasers (possibly frequency doubled, tripled, quadrupled or in other ways frequency manipulated), both pulsed and unpulsed, and (vi) light emitting diodes in the UV and visible area, both pulsed and unpulsed.

An optimal irradiation period and light intensity can easily be found by the skilled person by routine experiments. For practical reasons (e.g. in the large scale production of the medical device), the irradiation period should preferably not exceed 600 sec and in particular should not exceed 300 sec.

Currently the most preferred embodiments of the method of the present invention include:
I. A method for the preparation of a medical device element, said method comprising the steps of:
   (i) providing a thermoplastic substrate polymer;
   (ii) providing the coating composition;
   (iii) co-extruding the coating composition of step (ii) and the thermoplastic substrate polymer of step (i) so as to provide the medical device element of said substrate polymer having thereon a layer of said coating composition;
   (iv) irradiating the coating composition with UV or visible light so as to covalently cross-link said coating composition.
II. A method for the preparation of a medical device element, said method comprising the steps of:
   (i) providing a prefabricated shaped article and optionally a thermoplastic substrate polymer;
   (ii) providing a coating composition;
   (iii) co-extruding the coating composition of step (ii) on the prefabricated shaped article and, if present, the thermoplastic substrate polymer of step (i) so as to provide the medical device element of said prefabricated shaped article and, if present, said substrate polymer having thereon a layer of said coating composition, wherein, when said substrate polymer is present, said prefabricated shaped article has thereon a layer of said substrate polymer;
   (iv) irradiating the coating composition with UV or visible light so as to covalently cross-link said coating composition.
III. A method for the preparation of a medical device element, said method comprising the steps of:
   (i) providing a thermoplastic substrate polymer;
   (ii) providing a coating composition;
   (iii) injection moulding the coating composition of step (ii) and the thermoplastic substrate polymer of step (i) so as to provide the medical device element of said substrate polymer having thereon a layer of said coating composition;
   (iv) irradiating the coating composition with UV or visible light so as to covalently cross-link said coating composition.
IV. A method for the preparation of a medical device element, said method comprising the steps of:
   (i) providing a prefabricated shaped article and optionally a thermoplastic substrate polymer;
   (ii) providing a coating composition;
   (iii) injection moulding the coating composition of step (ii) on the prefabricated shaped article and, if present, the thermoplastic substrate polymer of step (i) so as to provide the medical device element of said prefabricated shaped article and, if present, said substrate polymer having thereon a layer of said coating composition, wherein, when said substrate polymer is present, said prefabricated shaped article has thereon a layer of said substrate polymer;
   (iv) irradiating the coating composition with UV or visible light so as to covalently cross-link said coating composition.

### Novel medical devices

It is believed that the medical device elements selected from tubes and catheters resulting from the method described above represent products which are novel *per se.* Such medical devices are i.a. characterised by the residues of photo-initiator moieties and such residues constitute 0.01-20 % by weight of the combined amount of the one or more hydrophilic polymer(s) and the one or more low molecular weight scaffolds.

When used herein, the term "residues of photo-initiator moieties" means the photo-initiator moieties in the form existing after the photo-initiator moieties have conducted the desired action, i.e. to facilitate - either directly or indirectly - the cross-linking of the coating composition, in particular the cross-linking of the chains of the poly(ethylene oxide)(s) and any non-thermoplastic hydrophilic polymers. The residues of the photo-initiator moieties are typically recognized as forms which are rearranged or cleaved at the molecular level compared to the native photo-initiator.

The content of residues of photo-initiator moieties in the coating can likely be determined from NMR (solution or solid state) spectroscopy, as the photo-initiator gives rise to resonances in the aromatic region of the spectrum whereas a hydrophilic polymer such as PEO has resonances in the aliphatic region. Integrated intensities obtained from e.g. a 1 H-NMR spectrum can be used to determine the content of the photo-initiator relative to other species in the coating. Alternatively, from the elemental analysis and/or XPS analysis a sum-formula of the coating can be deduced, which can be used directly to determine the content of the photo-initiator in the coating. Yet another method is to use the intensity of distinct bands in UV-vis, IR and/or NIR spectra of both the photo-initiator and the other species and entities in the coating. By evaluating the relative intensities, the photo-initiator content can be determined. Chromatography techniques such as HPLC, SEC and LC-MSn may also be used to determine the content of photo-initiator present in a coating by comparing integrated intensities from the chromatograms. In LC-MSn, mass-spectrometry is used to identify the origin of the signals (e.g. from the photo-initiator) in the chromatogram. In for example SEC, additional experiments such as NMR are needed to further identify the origin of each signal observed in the chromatogram. In addition, GC-MS techniques may be used similar to LC-MS techniques but with additional needed standards and calibrations prior to analyzing the actual coating composition. Chemical derivatization of the photo-initiator and/or other species and entities in the coating prior to utilizing the analytical techniques described above may be necessary. Atomic absorption measurements also provide an analytical tool for determining the composition of a coating. In principle any spectroscopic and/or spectrometric technique, where distinct integrated signals can be assigned to a specific chemical functionality and relative abundance can be used to determine the relative amount of photo-initiator present in the coating. Prior to determining the relative amount of photo-initiator in a coating, some experiments should be performed summarized in the following:
1. Degradation of the photo-initiator should be documented both as a result of heat and UV-vis radiation and relevant combinations thereof. Such degradation information may be used to determine the amount of photo-initiator present in the coating prior to exposing the coating to curing.
2. Diffusion of the photo-initiator present in the coating into a surrounding medium. More specifically, diffusion into an aqueous or highly polar medium as a function of time of one or more photo-initiators present in a coating should be documented. Additionally, diffusion into non-polar media should be documented. Given a hydrophilic coating contained in a medium and the amount of time the coating has been contained, such diffusion data may be used to determine the amount of photo-initiator present in the coating prior to containment.

By having such degradation and diffusion data at hand, it is possible to determine the relative amount of residues of photo-initiator moieties present in a coating prior to processing conditions.

Hence, the present invention also relates to medical devices comprising a medical device element of a thermoplastic substrate polymer having thereon a layer of a covalently cross-linked coating composition of (a) one or more hydrophilic polymer(s) optionally in combination with one or more additional polymers, said one or more hydrophilic polymer(s) constituting at least 50 % by weight of said polymer constituent(s), and (b) one or more low molecular weight scaffolds having a plurality of residues of photo-initiator moieties, wherein the residues of photo-initiator moieties constitute 0.01-20 % by weight of the combined amount of the one or more hydrophilic polymer(s), any additional polymers and the one or more low molecular weight scaffolds; wherein said coating composition is (co)extruded or injection moulded with said thermoplastic substrate polymer; and wherein the covalent cross-linking of the coating composition is the result of the presence of one or more photo-initiators in the coating composition, said photo-initiator moieties being covalently linked to the low molecular weight scaffold and/or being covalently incorporated into the backbone of the low molecular weight scaffold, and the exposure of the coating composition to UV or visible light; wherein the thermoplastic substrate polymer comprises a polymer selected from the group of:
Ethylene (Meth)acrylic Acid copolymer
Ethylene - (Meth)acrylic Acid - Acrylic ester - terpolymer
Ethylene (Meth)acrylic Acid ionomer, and
wherein the medical device element is selected from tubes and catheters.

The present invention further relates to a novel medical device comprising a medical device element of a prefabricated shaped article having thereon a layer of a covalently cross-linked coating composition of (a) as the only polymer constituent(s), one or more hydrophilic polymer(s), and (b) one or more low molecular weight scaffolds having a plurality of residues of photo-initiator moieties, wherein the residues of photo-initiator moieties constitute 0.01-20 % by weight of the combined amount of the one or more hydrophilic polymer(s) and the one or more low molecular weight scaffolds; wherein said coating composition is extruded or injection moulded with said prefabricated shaped article; and wherein the covalent cross-linking of the coating composition is the result of one or more photo-initiators in the coating composition, said photo-initiator moieties being covalently linked to the low molecular weight scaffold and/or being covalently incorporated into the backbone of the low molecular weight scaffold, and the exposure of the coating composition to UV or visible light; wherein the prefabricated shaped article comprises a polymer selected from the group of:
Ethylene (Meth)acrylic Acid copolymer
Ethylene - (Meth)acrylic Acid - Acrylic ester - terpolymer
Ethylene (Meth)acrylic Acid ionomer, and
wherein the medical device element is selected from tubes and catheters.

The present invention still further relates to a novel medical device comprising a medical device element of a prefabricated shaped article having thereon a layer of a thermoplastic substrate polymer, where said thermoplastic substrate polymer has thereon a layer of a covalently cross-linked coating composition of (a) as the only polymer constituent(s), one or more hydrophilic polymer(s), and (b) one or more low molecular weight scaffolds having a plurality of residues of photo-initiator moieties, wherein the residues of photo-initiator moieties constitute 0.01-20 % by weight of the combined amount of the one or more hydrophilic polymer(s) and the one or more low molecular weight scaffolds; wherein said coating composition is (co)extruded or injection moulded with said prefabricated shaped article and said thermoplastic substrate polymer; and wherein the covalent cross-linking of the coating composition is the result of the presence of one or more photo-initiators in the coating composition, said photo-initiator moieties being covalently linked to the low molecular weight scaffold and/or being covalently incorporated into the backbone of the low molecular weight scaffold, and the exposure of the coating composition to UV or visible light; wherein the prefabricated shaped article and/or the thermoplastic substrate polymer comprises a polymer selected from the group of:
Ethylene (Meth)acrylic Acid copolymer
Ethylene - (Meth)acrylic Acid - Acrylic ester - terpolymer
Ethylene (Meth)acrylic Acid ionomer, and
wherein the medical device element is selected from tubes and catheters.

The materials useful as the prefabricated shaped article, the thermoplastic substrate polymer and as constituents of the coating compositions are as described above for the method of the invention.

Hence, in one embodiment, the thermoplastic substrate polymer is selected from the group consisting of Ethylene (Meth)acrylic Acid copolymer, Ethylene - (Meth)acrylic Acid - Acrylic ester - terpolymer, and Ethylene (Meth)acrylic Acid ionomer.

In a further embodiment, the hydrophilic polymer is selected from the group consisting of poly(vinyl lactams) [e.g. PVP], PEO, polyoxazolines, PVOH, and polyacrylates. The currently most preferred hydrophilic polymer is PEO.

### EXAMPLES

### Materials

PEO was 1 NF pharmaceutical grade from Sumitomo, JP.

Bomar oligomer photoinitiator was custom synthesized by Bomar Specialties Co (Winsted, CT) and distributed in Europe by IGM Resins (Waalwijk, the Netherlands). It was an aliphatic, trifunctional polyether urethane of medium molecular weight, which was functionalised with Irgacure 2959 at all three ends. The content of Irgacure 2959 in Compound 4 was 33.0 w/w-%, as indicated by Bomar. The compound did not contain any acrylate groups.

SEBS compound was Versaflex HC 226 from GLS Corporation.

EMA's were Lotryl 20MA08 from Arkema and Elvaloy 1224 AC from DuPont.

EEA was Elvaloy 2112 AC from DuPont.

EAA's were Nucrel 31001 and Nucrel 3990 E from DuPont and Primacor 1410, Primacor 1430 and Primacor 3460 from DuPont.

EMAA's were Nucrel 903 HC, Nucrel 1202 HC and Nucrel 925.

A-EMA terpolymers were Lucalen A2910 and Lucallen A3110 from LyondeII-BaseII.

*The coating composition for all examples was as follows*

| Ingredients | Compound A |
|---|---|
| PEO 1NF from Sumitomo | 98.0 % |
| Irgacure 2959 bound to aliphatic, hydrophobic polyurethanes | 1.5 % |
| Irganox 1010 antioxidant | 0,5 % |

All percentages and parts given are weight/weight-% unless otherwise stated.

These ingredients were compounded in a twin-screw extruder. The ingredients were fed to the extruder by gravimetric feeders, extruded into strands and pelletized.

In the co-extrusion coating process, two single screw extruders were connected to a two layer co-extrusion crosshead with a draw down type die. Extruder #1 was charged with the substrate/tie layer and extruder #2 was charged with Compound A. The two layers were extruded onto a prefabricated tube of the actual polyolefin material to form hydrophilic coated tubing. Standard process speed was 20 m/min, but a speed up to 40m/min was tried in some cases. In order to optimize the processing, also inline UV lamp (Fusion 600l H-lamp) intensity was varied between 60 and 100 % intensity. The ratio of inner to outer layer was varied by adjusting the output of either extruder by increasing or lowering the screw speed. The thickness of the layers was adjusted by varying either the output or the haul-off speed.

The two extruders had the same temperature profile.

| Extruder Zone | Zone 1 | Zone 2 | Zone 3 | Zone 4 | Zone 5 | Head | Die |
|---|---|---|---|---|---|---|---|
| Temp (degC) | 35 | 60 | 130 | 140 | 150 | 170 | 175 |

After extrusion, the coated tube was cut into 35 cm long test samples.

### Evaluation of trials

Before evaluation the samples were packed in Aluminium pouches with 6 ml 6%PEG2000 in water per CH12 male catheter (35 cm length) and E-beam sterilized 2x27 kGy before evaluation.

### Friction measurement

The swelled and sterilized catheter was unpacked and immediately thereafter the slippery part was placed horizontally between a lower and an upper polished block of stainless steel in such a way that the upper block exerted its full gravitational force on the catheter. The mass and length of the upper steel block was 266 g and 34 mm, respectively. The steel blocks were moved back and forth by a motor and the push and pull force was measured continuously by a load cell attached to the connector of the catheter. The initial push/pull force was averaged and the friction force reported was the average of determinations on three separate catheters. A good catheter should have a small friction force, preferentially below 100 mN.

### Friction measurement after 10 min. drying

The swelled and sterilized catheter was unpacked and hanged up vertically in a climate room at 25°C and 50% relative humidity. Immediately thereafter the slippery part was placed horizontally between a lower and an upper polished block of stainless steel in such a way that the upper block exerted its full gravitational force on the catheter. The mass and length of the upper steel block were 266 g and 34 mm, respectively. The steel blocks were moved back and forth by a motor and the push and pull force was measured continuously by a load cell attached to the connector of the catheter. The initial push/pull force was averaged and the friction force reported was the average of determinations on three separate catheters. A good catheter should have a small friction force after 10 min. drying, preferentially below 150 mN.

### Subjective evaluation of adhesion

The coating adhesion between the layers (preshaped article to substrate/tie layer and substrate/tie layer to coating) was given a score from 1 to 5
1. Complete delaminating
2. Partly delaminating
3. Poor adhesion
4. Good adhesion
5. Very good adhesion

### Subjective evaluation of smoothness and subjective friction

The smoothness and subjective friction of the gels were scored on a subjective scale from 1 to 5:
1. Very rough
2. Rough
3. Rather rough but rather low subjective friction
4. Almost smooth and low subjective friction
5. Smooth and low subjective friction

### Subjective evaluation of anti-kinking properties

The anti-kink properties were scored on a subjective scale from 1 to 5:
1. Easy kinking as standard polyolefin, e.g LDPE
2. Better than standard flexible polyolefin, VLDPE
3. Acceptable kinking
4. As good as TPU Estane 58212
5. As good as plasticized PVC

### Subjective evaluation of catheter stiffness

The catheter stiffness was scored on the following subjective scale:
A. Unacceptable low
B. As catheter in plasticized PVC (soft and gentle)
C. Medium stiffness
D. As SpeediCath® catheter in Estane 58212 TPU (good control of insertion)
E. Slightly higher than SpeediCath
F. Unacceptable high

### Example 1: Coating on tube made of EAA

### Trials

| No. | Tube material | Tie-layer material | Tie-layer extruder | Coating extruder | Coating speed | Relative UV intensity % |
|---|---|---|---|---|---|---|
| 1.1* | Primacor 1410 | Estane 58311 | 20 rpm | 15 rpm | 15 m/min | 80 |
| 1.2* | Primacor 1410 | Primacor 3460 | 20 rpm | 15 rpm | 15 m/min | 80 |
| 1.3* | Primacor 1410 | Primacor 3460 | 20 rpm | 30 rpm | 15 m/min | 80 |
| 1.4* | Primacor 1410 | Lotryl 20 MA08 | 20 rpm | 30 rpm | 15 m/min | 80 |
| 1.5* | Primacor 1410 | Nucrel 925 | 20 rpm | 30 rpm | 15 m/min | 80 |
| 1.6** | Nucrel 31001 | Nucrel 3990 E | 20 rpm | 20 rpm | 20 m/min | 80 |
| 1.7** | Nucrel 31001 | Nucrel 925 | 20 rpm | 20 rpm | 20 m/min | 80 |

| | | | | | | |
|---|---|---|---|---|---|---|
| *) *Coating recipe for trial 1.1 to 1.5 is with industrial grade Sumitomo PEO-1Z instead of pharmaceutical grade Sumitomo PEO 1 NF.* **) *Packaged with 6% PVP swelling media instead of 6% PEG2000.* | | | | | | |

### Results

| No. | Friction 0 min | Friction 10 min | Coating adhesion | Coating smoothness | Subjective anti-kink | Subjective stiffness |
|---|---|---|---|---|---|---|
| 1.1* | 51 mN | 177 mN | 5 | 4 | 4 | E |
| 1.2* | 54 mN | 259 mN | 5 | 4 | 4 | E |
| 1.3* | 58 mN | 105 mN | 5 | 4 | 4 | E |
| 1.4* | 64 mN | 74 mN | 5 | 4 | 4 | E |
| 1.5* | 54 mN | 101 mN | 5 | 4 | 4 | E |
| 1.6 | 51 mN | 54 mN | 5 | 5 | 4 to 5 | D |
| 1.7 | 41 mN | 55 mN | 5 | 5 | 4 to 5 | D |

### Discussion

The results show that EAA gives good anti-kink properties for the tube and additionally works as a substrate/ tie-layer giving good adhesion to the PEO coating. Especially good anti-kink results are obtained with Nucrel 31001, but still Primacor 1410 is equally good as TPU Estane 58212. The results show that EAA (Nucrel 3990 E), EMAA (Nucrel 925), EMA (Lotryl 20MA08) and TPU (Estane 58212) all give good adhesion as well to the EAA tube as to the PEO coating. The results for friction after 10 min. and for coating smoothness are better for trial 1.6 to 1.7 than for trial 1.1 to 1.5. This is likely to be because trials 1.1 to 1.5 were made with industrial grade PEO as described above.

### Example 2: Coating by co-extrusion on tube made of EMAA

### Trials

| No. | Tube material | Tie-layer material | Tie-layer extruder | Coating extruder | Coating speed | Relative UV intensity % |
|---|---|---|---|---|---|---|
| 2.1 | Nucrel 903 | Nucrel 925 | 20 rpm | 20 rpm | 20 m/min | 100 |
| 2.2 | Nucrel 903 | Nucrel 903 HC | 20 rpm | 20 rpm | 20 m/min | 100 |
| 2.3 | Nucrel1202H | Nucrel 903 HC | 20 rpm | 20 rpm | 20 m/min | 100 |
| 2.4 | Nucrel1202H | Nucrel 925 | 20 rpm | 20 rpm | 20 m/min | 100 |

### Results

| No. | Friction 0 min | Friction 10 min | Coating adhesion | Coating smoothness | Subjective anti-kink | Subjective stiffness |
|---|---|---|---|---|---|---|
| 2.1 | 66 mN | 77 mN | 5 | 5 | 4 | E |
| 2.2 | 59 mN | 66 mN | 5 | 5 | 4 | E |
| 2.3 | 59 mN | 57 mN | 5 | 5 | 4 | E |
| 2.4 | 56 mN | 92 mN | 5 | 5 | 4 | E |

### Discussion

The results show that EMAA gives good anti-kink properties for the tube. The results show that at least EMAA (Nucrel 925) and EMA (Lotryl 20MA08) give good adhesion to the EMAA tube and to the PEO coating. The catheters are slightly stiffer than SpeediCath catheters made in Estane 58212.

### Example 3: Coating by co-extrusion on tube made of EMA

### Trials

| No. | Tube material | Tie-layer material | Tie-layer extruder | Coating extruder | Coating speed | Relative UV intensity % |
|---|---|---|---|---|---|---|
| 3.1* | Elvaloy1224AC | Nucrel 925 | 20 rpm | 20 rpm | 20 m/min | 80 |
| 3.2* | Elvaloy1224AC | Nucrel 925 | 20 rpm | 20 rpm | 20 m/min | 100 |

| | | | | | | |
|---|---|---|---|---|---|---|
| ***) Packaged with 6% PVP swelling media instead of 6% PEG2000.* | | | | | | |

### Results

| No. | Friction 0 min | Friction 10 min | Coating adhesion | Coating smoothness | Subjective anti-kink | Subjective stiffness |
|---|---|---|---|---|---|---|
| 3.1 | 55 mN | 78 mN | 3 | 4 | 3 to 4 | B |
| 3.2 | 48 mN | 71 mN | 4 | 4 | 3 to 4 | B |

### Discussion

The results show that a soft EMA in combination with a harder EMAA co-polymer as the substrate/tie layer gives good anti-kink properties for the tube substrate/tie-layer. This demonstrates that a tube, soft as plasticized PVC, but still with very good anti-kink properties can be made by this combination.

### Example 4: Coating by co-extrusion on tube made of EEA

### Trials

| No. | Tube material | Tie-layer material | Tie-layer extruder | Coating extruder | Coating speed | Relative UV intensity % |
|---|---|---|---|---|---|---|
| 4.1 | Elvaloy2112AC | Elvaloy2715AC | 20 rpm | 20 rpm | 20 m/min | 100 |
| 4.2 | Elvaloy2112AC | Nucrel 925 | 20 rpm | 20 rpm | 20 m/min | 100 |
| 4.3 | Elvaloy2112AC | Nucrel 925 | 70 rpm | 20 rpm | 20 m/min | 100 |
| 4.4 | Elvaloy2116AC | Nucrel 925 | 20 rpm | 20 rpm | 20 m/min | 100 |
| 4.5 | Elvaloy2116AC | Nucrel 925 | 70 rpm | 20 rpm | 20 m/min | 100 |

### Results

| No. | Friction 0 min | Friction 10 min | Coating adhesion | Coating smoothness | Subjective anti-kink | Subjective stiffness |
|---|---|---|---|---|---|---|
| 4.1 | NA | NA | 2 | NA | 3 | C |
| 4.2 | 64 mN | 101 mN | 5 | 5 | 3 | C |
| 4.3 | 54 mN | 64 mN | 5 | 5 | 4 | C to D |
| 4.4 | 58 mN | 76 mN | 4 | 4 | 3 | B to C |
| 4.5 | 58 mN | 68 mN | 5 | 4 | 4 | C |

### Discussion

The results show that EEA gives rather good anti-kink properties for the tube when compared to standard polyolefin's like LDPE and VLDPE. It can be seen that EEA does not work as a substrate/ tie-layer towards the PEO coating. However, it is shown that EMAA (Nucrel 925) gives good adhesion to as well the EEA tube as to the PEO coating. By using a rather thick layer of EMAA (and likely also EAA). the anti-kink properties can (as an added benefit) be improved to equal TPU Estane 58212. However, this also gives more stiffness to the tubing as compared to the pure EEA.

### Example 5: Coating by co-extrusion on tube of EAA-EMA terpolymer

### Trials

| No. | Tube material | Tie-layer material | Tie-layer extruder | Coating extruder | Coating speed | Relative UV intensity % |
|---|---|---|---|---|---|---|
| 5.1 | LucalenA2910M | Nucrel 925 | 20 rpm | 20 rpm | 20 m/min | 80 |
| 5.2 | LucalenA2910M | Nucrel 925 | 20 rpm | 20 rpm | 20 m/min | 100 |

### Results

| No. | Friction 0 min | Friction 10 min | Coating adhesion | Coating smoothness | Subjective anti-kink | Subjective stiffness |
|---|---|---|---|---|---|---|
| 5.1 | NA | NA | 1 | NA | 3 | B to C |
| 5.2 | 60 mN | 87 mN | 5 | 5 | 3 | B to C |

### Discussion

The results show that EMA-EAA ter-polymer gives rather good anti-kink properties for the tube when compared to standard polyolefins like LDPE and VLDPE. At least EMAA (Nucrel 925) can be used as a substrate/ tie-layer, but it requires rather high UV intensity to get a good coating adhesion. More substrate/tie-layers can also be used. For instance the EMA-EAA ter-polymer could be used as tube material co-extruded with a skin layer of e.g. EMAA. On this prefabricated article a two-layer combination of e.g. EMAA substrate/tie layer and the PEO coating could successively be co-extruded.

### Example 6: Coating by co-extrusion on tube made of SEBS compound

### Trials

| No. | Tube material | Tie-layer material | Tie-layer extruder | Coating extruder | Coating speed | Relative UV intensity % |
|---|---|---|---|---|---|---|
| 5.1 | VersaflexHC226 | Nucrel 925 | 20 rpm | 20 rpm | 20 m/min | 80 |
| 5.2 | VersaflexHC226 | Nucrel 925 | 20 rpm | 20 rpm | 20 m/min | 100 |

### Results and discussion

| No. | Friction 0 min | Friction 10 min | Coating adhesion | Coating smoothness | Subjective anti-kink | Subjective stiffness |
|---|---|---|---|---|---|---|
| 5.1 | 135 mN | 107 mN | 1 | 4 | 3 | B to C |
| 5.2 | 68 mN | 78 mN | 3 to 4 | 3 to 4 | 3 | B to C |

### Discussion

The results show that a commercial SEBS compound can give rather good anti-kink properties for the tube when compared to standard polyolefins like LDPE and VLDPE. At least EMAA (Nucrel 925) can to some extend be used as a substrate/ tie-layer, but a totally good result was not obtained even with very high UV intensity.

### Overall discussion of Examples 1 to 6

The overall results of the examples above show the range of polyolefin co-polymers identified in this work optionally in combination with other polymers in a multi layer construction can give hydrophilic coated catheters with mechanical properties as stiffness and anti-kink that cover the whole span of commercial intermittent urinary catheters ranging from soft plasticized PVC catheters to catheters like SpeediCath, which are in the stiffer and more controllable range.

The softer material combinations, which cover a stiffness range of soft PVC, can include acrylate co-polymers like EMA, EAA, EnBA with an acrylate copolymer content ranging from 10 to 30% or ter-polymers of polyethylene in combination with acrylic or methacrylic acid co-polymers. The acrylate co-polymers can be used either as tube material with an EAA or EMAA polymer as the substrate/tie layer, or as a substrate/tie layer on a tube made out of EAA or EMAA co-polymers. EMA with a rather high acrylate content, 20 to 24%, is one preferred copolymer for the combinations, either when used as tie layer and in the situation where it is used as tube material. Also styrene block copolymer compounds can be used for a soft tube in combination with EAA or EMAA as the substrate/tie layer.

The harder materials, which cover stiffness of SpeediCath TPU catheter, are co-polymers of polyethylene with acrylic acid or methacrylic acid with an acrylate co-polymer content ranging from 5 to 30%, preferentially between 8 and 15%. They can also be lonomers of the same co-polymers. They can be used alone or they can be combined with other polymers like acrylic ester co-polymers or polyurethane's in a multi layer construction to obtain the desired properties like catheter stiffness, anti-kink properties and coating adhesion.

The good mechanical properties for a catheter, which has been found in the examples, cannot be obtained with standard polyolefins like LDPE and VLDPE.

It has also been found in the examples that many of the materials work as a substrate/ tie-layer towards the UV curable PEO coating. Especially good for this are EAA and EMAA copolymers.

It has also been shown in the examples that the materials can be combined in different layer configurations and thickness of the layers in order to obtain and optimise certain properties for the catheter like catheter stiffness, anti-kink properties and coating adhesion.

## Claims

1. A method for the preparation of a medical device element, said method comprising the steps of:
(i) providing a prefabricated shaped article and/or one or more layers of a thermoplastic substrate polymer;
(ii) providing a coating composition comprising:
(a) as the only polymer constituent(s), one or more hydrophilic polymer(s), and
(b) one or more low molecular weight scaffolds having a plurality of photo-initiator moieties covalently linked thereto and/or covalently incorporated therein,
wherein the photo-initiator moieties constitute 0.01-20 % by weight of the combined amount of the hydrophilic polymer(s) and the one or more low molecular weight scaffolds;
(iii) extruding, injection moulding or powder coating the coating composition of step (ii) on the prefabricated shaped article and/or the thermoplastic substrate polymer of step (i) so as to provide the medical device element of said prefabricated shaped article and/or said substrate polymer having thereon a layer of said coating composition, wherein, when both of said prefabricated shaped article and said substrate polymer are present, said prefabricated shaped article has thereon a layer of said substrate polymer;
(iv) irradiating the coating composition with UV or visible light so as to covalently cross-link said coating composition;
wherein the prefabricated shaped article and/or one or more of the thermoplastic substrate polymers of step (i) comprising a polymer selected from the group of:
Ethylene (Meth)acrylic Acid copolymer
Ethylene - (Meth)acrylic Acid - Acrylic ester - terpolymer
Ethylene (Meth)acrylic Acid ionomer; and
wherein the medical device element is selected from tubes and catheters.

2. The method according to claim 1, wherein the hydrophilic polymer is a poly(ethylene oxide).

3. The method according to any one of the preceding claims, wherein the scaffold is selected from polyethylene glycols, poly(styrene-co-maleic anhydride)s, aliphatic polyether urethanes, polyetheramines, and polyesters.

4. The method according to any one of the preceding claims, wherein the weight average molecular weight of the scaffold is in the range of 100-10,000 Da (g/mol).

5. The method according to any one of the preceding claims, wherein the coating composition consists of
20-99.99 % by weight of the one or more hydrophilic polymer(s),
0-10 % by weight of one or more plasticizers,
0.01-80 % by weight of the one or more low molecular weight scaffolds, and
0-5 % by weight of other components.

6. The method according to any one of the preceding claims, wherein a prefabricated shaped article is provided in step (i), and wherein step (iii) involves extruding, injection moulding or powder coating the coating composition of step (ii) on the prefabricated shaped article of step (i) so as to provide the medical device element of said prefabricated shaped article having thereon a layer of said coating composition.

7. The method according to any one of the claims 1-5, wherein a thermoplastic substrate polymer is provided in step (i), and wherein step (iii) involves extruding or injection moulding the coating composition of step (ii) together with the thermoplastic substrate polymer of step (i) so as to provide the medical device element of said thermoplastic substrate polymer having thereon a layer of said coating composition.

8. The method according to any one of the claims 1-5, wherein a prefabricated shaped article and a thermoplastic substrate polymer are provided in step (i), and wherein step (iii) involves extruding, injection moulding or powder coating the coating composition of step (ii) on the prefabricated shaped article together with the thermoplastic substrate polymer of step (i) so as to provide the medical device element of said prefabricated shaped article and said thermoplastic substrate polymer, said prefabricated shaped article having thereon a layer of said thermoplastic substrate polymer and said thermoplastic substrate polymer having thereon a layer of said coating composition.

9. The method according to any one of the claims 1-5 and 7-8, wherein the medical device element comprises one layer of a thermoplastic substrate polymer.

10. The method according to any one of the claims 1-5 and 7-8, wherein the medical device element comprises two layers of a thermoplastic substrate polymer.

11. The method according to any one of the preceding claims, wherein the medical device element is a catheter.

12. The method according to any one of the preceding claims, wherein the medical device element is a urinary catheter.

13. A medical device comprising a medical device element of a thermoplastic substrate polymer having thereon a layer of a covalently cross-linked coating composition of
(a) as the only polymer constituent(s), one or more hydrophilic polymer(s), and
(b) one or more low molecular weight scaffolds having a plurality of residues of photo-initiator moieties, wherein the residues of photo-initiator moieties constitute 0.01-20 % by weight of the combined amount of the one or more hydrophilic polymer(s) and the one or more low molecular weight scaffolds;
wherein said coating composition is (co)extruded or injection moulded with said thermoplastic substrate polymer; and wherein the covalent cross-linking of the coating composition is the result of the presence of one or more photo-initiators in the coating composition, said photo-initiator moieties being covalently linked to the low molecular weight scaffold and/or being covalently incorporated into the backbone of the low molecular weight scaffold, and the exposure of the coating composition to UV or visible light;
wherein the thermoplastic substrate polymer comprises a polymer selected from the group of:
Ethylene (Meth)acrylic Acid copolymer
Ethylene - (Meth)acrylic Acid - Acrylic ester - terpolymer
Ethylene (Meth)acrylic Acid ionomer; and
wherein the medical device element is selected from tubes and catheters.

14. A medical device comprising a medical device element of a prefabricated shaped article having thereon a layer of a covalently cross-linked coating composition of
(a) as the only polymer constituent(s), one or more hydrophilic polymer(s), and
(b) one or more low molecular weight scaffolds having a plurality of residues of photo-initiator moieties, wherein the residues of photo-initiator moieties constitute 0.01-20 % by weight of the combined amount of the one or more hydrophilic polymer(s) and the one or more low molecular weight scaffolds;
wherein said coating composition is extruded or injection moulded with said prefabricated shaped article; and wherein the covalent cross-linking of the coating composition is the result of one or more photo-initiators in the coating composition, said photo-initiator moieties being covalently linked to the low molecular weight scaffold and/or being covalently incorporated into the backbone of the low molecular weight scaffold, and the exposure of the coating composition to UV or visible light;
wherein the prefabricated shaped article comprises a polymer selected from the group of:
Ethylene (Meth)acrylic Acid copolymer
Ethylene - (Meth)acrylic Acid - Acrylic ester - terpolymer
Ethylene (Meth)acrylic Acid ionomer; and
wherein the medical device element is selected from tubes and catheters.

15. A medical device comprising a medical device element of a prefabricated shaped article having thereon a layer of a thermoplastic substrate polymer, where said thermoplastic substrate polymer has thereon a layer of a covalently cross-linked coating composition of
(a) as the only polymer constituent(s), one or more hydrophilic polymer(s), and
(b) one or more low molecular weight scaffolds having a plurality of residues of photo-initiator moieties, wherein the residues of photo-initiator moieties constitute 0.01-20 % by weight of the combined amount of the one or more hydrophilic polymer(s) and the one or more low molecular weight scaffolds;
wherein said coating composition is (co)extruded or injection moulded with said prefabricated shaped article and said thermoplastic substrate polymer; and wherein the covalent cross-linking of the coating composition is the result of the presence of one or more photo-initiators in the coating composition, said photo-initiator moieties being covalently linked to the low molecular weight scaffold and/or being covalently incorporated into the backbone of the low molecular weight scaffold, and the exposure of the coating composition to UV or visible light;
wherein the prefabricated shaped article and/or the thermoplastic substrate polymer comprises a polymer selected from the group of:
Ethylene (Meth)acrylic Acid copolymer
Ethylene - (Meth)acrylic Acid - Acrylic ester - terpolymer
Ethylene (Meth)acrylic Acid ionomer; and
wherein the medical device element is selected from tubes and catheters.

16. The medical device according to any one of claims 13-15, wherein the medical device element is a catheter.

17. The medical device according to any one of claims 13-15, wherein the medical device element is a urinary catheter.

## Patentansprüche

1. Verfahren zur Herstellung eines medizinischen Vorrichtungselements, wobei das Verfahren folgende Schritte umfasst:
(i) Bereitstellen eines vorgefertigten geformten Artikels und/oder einer oder mehrerer Schichten eines thermoplastischen Substratpolymers;
(ii) Bereitstellen einer Beschichtungszusammensetzung, umfassend:
(a) als einzige(n) Polymerbestandteil(e) ein oder mehrere hydrophile Polymere, und
(b) ein oder mehrere niedermolekulargewichtige Gerüste mit einer Vielzahl von Photoinitiator-anteilen, die kovalent damit verknüpft und/oder kovalent darin eingebaut sind, wobei die Photoinitiatoranteile 0,01 bis 20 Gew.% der kombinierten Menge des hydrophilen Polymers/der hydrophilen Polymere und des einen niedermolekulargewichtigen Gerüsts oder der mehreren niedermolekulargewichtigen Gerüste stellen;
(iii) Extrudieren, Spritzgießen oder Pulverbeschichten der Beschichtungszusammensetzung von Schritt (ii) auf den vorgefertigten geformten Artikel und/oder das thermoplastische Substratpolymer von Schritt (i), um so das medizinische Vorrichtungselement des vorgefertigten geformten Artikels und/oder des Substratpolymers mit einer Schicht der Beschichtungszusammensetzung darauf bereitzustellen, wobei, wenn sowohl der vorgefertigte geformte Artikel als auch das Substratpolymer vorhanden sind, der vorgefertigte geformte Artikel eine Schicht des Substratpolymers darauf aufweist;
(iv) Bestrahlen der Beschichtungszusammensetzung mit UV- oder sichtbarem Licht, um die Beschichtungszusammensetzung kovalent zu vernetzen;
wobei der vorgefertigte geformte Artikel und/oder ein oder mehrere der thermoplastischen Substratpolymere von Schritt (i) ein Polymer umfassen, das ausgewählt ist aus der Gruppe:
Ethylen-(Meth)acrylsäure-Copolymer
Ethylen-(Meth)acrylsäure-Acrylester-Terpolymer
Ethylen-(Meth)acrylsäure-Ionomer und
wobei das medizinische Vorrichtungselement ausgewählt ist aus Rohren und Kathetern.

2. Verfahren nach Anspruch 1, wobei das hydrophile Polymer Poly(ethylenoxid) ist.

3. Verfahren nach einem der vorhergehenden Ansprüche, wobei das Gerüst ausgewählt ist aus Polyethylenglykolen, Poly(styrol-co-maleinsäureanhydrid)en, aliphatischen Polyetherurethanen, Polyetheraminen und Polyestern.

4. Verfahren nach einem der vorhergehenden Ansprüche, wobei das durchschnittliche Molekulargewicht (Gewichtsmittel) des Gerüsts im Bereich von 100 bis 10.000 Da (g/mol) liegt.

5. Verfahren nach einem der vorhergehenden Ansprüche, wobei die Beschichtungszusammensetzung besteht aus:
20 bis 99,99 Gew.% des einen oder der mehreren hydrophilen Polymere,
0 bis 10 Gew.% von einem oder mehreren Plastifizierungsmitteln,
0,01 bis 80 Gew.% des einen oder der mehreren niedermolekulargewichtigen Gerüste und
0 bis 5 Gew.% von anderen Komponenten.

6. Verfahren nach einem der vorhergehenden Ansprüche, wobei in Schritt (i) ein vorgefertigter geformter Artikel bereitgestellt wird und wobei Schritt (iii) Extrudieren, Spritzgießen oder Pulverbeschichten der Beschichtungszusammensetzung von Schritt (ii) auf den vorgefertigten geformten Artikel von Schritt (i) umfasst, um so das medizinische Vorrichtungselement des vorgefertigten geformten Artikels mit einer Schicht der Beschichtungszusammensetzung darauf bereitzustellen.

7. Verfahren nach einem der Ansprüche 1 bis 5, wobei in Schritt (i) ein thermoplastisches Substratpolymer bereitgestellt wird und wobei Schritt (iii) Extrudieren oder Spritzgießen der Beschichtungszusammensetzung von Schritt (ii) zusammen mit dem thermoplastischen Substratpolymer von Schritt (i) umfasst, um so das medizinische Vorrichtungselement des thermoplastischen Substratpolymers mit einer Schicht der Beschichtungszusammensetzung darauf bereitzustellen.

8. Verfahren nach einem der Ansprüche 1 bis 5, wobei in Schritt (i) ein vorgefertigter geformter Artikel und ein thermoplastisches Substratpolymer bereitgestellt werden und wobei Schritt (iii) Extrudieren, Spritzgießen oder Pulverbeschichten der Beschichtungszusammensetzung von Schritt (ii) auf den vorgefertigten geformten Artikel zusammen mit dem thermoplastischen Substratpolymer von Schritt (i) umfasst, um so das medizinische Vorrichtungselement des vorgefertigten geformten Artikels und des thermoplastischen Substratpolymers bereitzustellen, wobei der vorgefertigte geformte Artikel eine Schicht des thermoplastischen Substratpolymers darauf aufweist und das thermoplastische Substratpolymer eine Schicht der Beschichtungszusammensetzung darauf aufweist.

9. Verfahren nach einem der Ansprüche 1 bis 5 und 7 bis 8, wobei das medizinische Vorrichtungselement eine Schicht eines thermoplastischen Substratpolymers umfasst.

10. Verfahren nach einem der Ansprüche 1 bis 5 und 7 bis 8, wobei das medizinische Vorrichtungselement zwei Schichten eines thermoplastischen Substratpolymers umfasst.

11. Verfahren nach einem der vorhergehenden Ansprüche, wobei das medizinische Vorrichtungselement ein Katheter ist.

12. Verfahren nach einem der vorhergehenden Ansprüche, wobei das medizinische Vorrichtungselement ein Blasenkatheter ist.

13. Medizinische Vorrichtung, die ein medizinisches Vorrichtungselement aus einem thermoplastischen Substratpolymer mit einer Schicht einer kovalent vernetzten Beschichtungszusammensetzung darauf aufweist, die Folgendes umfasst:
(a) als einzige(n) Polymerbestandteil(e) ein oder mehrere hydrophile Polymere, und
(b) ein oder mehrere niedermolekulargewichtige Gerüste mit einer Vielzahl von Resten von Photoinitiatoranteilen, wobei die Reste der Photoinitiatoranteile 0,01 bis 20 Gew.% der kombinierten Menge des hydrophilen Polymers/der mehreren hydrophilen Polymere und des einen niedermolekulargewichtigen Gerüsts oder der mehreren niedermolekulargewichtigen Gerüste stellen;
wobei die Beschichtungszusammensetzung mit dem thermoplastischen Substratpolymer (co)extrudiert oder spritzgegossen ist; und wobei das kovalente Vernetzen der Beschichtungszusammensetzung das Ergebnis der Anwesenheit von einem oder mehreren Photoinitiatoren in der Beschichtungszusammensetzung ist, wobei die Photoinitiatoranteile kovalent mit dem niedermolekulargewichtigen Gerüst verknüpft und/oder kovalent in das Grundgerüst des niedermolekulargewichtigen Gerüsts eingebaut sind, und UV- oder sichtbares Licht auf die Beschichtungszusammensetzung einwirken gelassen wird; wobei das thermoplastische Substratpolymer ein Polymer ausgewählt aus der folgenden Gruppe umfasst:
Ethylen-(Meth)acrylsäure-Copolymer
Ethylen-(Meth)acrylsäure-Acrylester-Terpolymer
Ethylen-(Meth)acrylsäure-Ionomer und
wobei das medizinische Vorrichtungselement ausgewählt ist aus Rohren und Kathetern.

14. Medizinische Vorrichtung, die ein medizinisches Vorrichtungselement aus einem vorgefertigten geformten Artikel mit einer Schicht einer kovalent vernetzten Beschichtungszusammensetzung darauf aufweist, die Folgendes umfasst:
(a) als einzige(n) Polymerbestandteil(e) ein oder mehrere hydrophile Polymere, und
(b) ein oder mehrere niedermolekulargewichtige Gerüste mit einer Vielzahl von Resten von Photoinitiatoranteilen, wobei die Reste der Photoinitiatoranteile 0,01 bis 20 Gew.% der kombinierten Menge des hydrophilen Polymers/der mehreren hydrophilen Polymere und des einen niedermolekulargewichtigen Gerüsts oder der mehreren niedermolekulargewichtigen Gerüste stellen;
wobei die Beschichtungszusammensetzung mit dem vorgefertigten geformten Artikel extrudiert oder spritzgegossen ist; und
wobei die kovalente Vernetzung der Beschichtungszusammensetzung das Ergebnis von einem oder mehreren Photoinitiatoren in der Beschichtungszusammensetzung ist, wobei die Photoinitiatoranteile kovalent mit dem niedermolekulargewichtigen Gerüst verknüpft und/oder kovalent in das Grundgerüst des niedermolekulargewichtigen Gerüsts eingebaut sind, und die Beschichtungszusammensetzung UV- oder sichtbarem Licht ausgesetzt wird;
wobei der vorgefertigte geformte Artikel ein Polymer ausgewählt aus der folgenden Gruppe umfasst:
Ethylen-(Meth)acrylsäure-Copolymer
Ethylen-(Meth)acrylsäure-Acrylester-Terpolymer
Ethylen-(Meth)acrylsäure-Ionomer und
wobei das medizinische Vorrichtungselement ausgewählt ist aus Rohren und Kathetern.

15. Medizinische Vorrichtung, die ein medizinisches Vorrichtungselement aus einem vorgefertigten geformten Artikel mit einer Schicht eines thermoplastischen Substratpolymers darauf umfasst, wobei das thermoplastische Substratpolymer eine Schicht aus einer kovalent vernetzten Beschichtungszusammensetzung darauf aufweist, welche umfasst:
(a) als einzige(n) Polymerbestandteil(e) ein oder mehrere hydrophile Polymere, und
(b) ein oder mehrere niedermolekulargewichtige Gerüste mit einer Vielzahl von Resten von Photoinitiatoranteilen, wobei die Reste der Photoinitiatoranteile 0,01 bis 20 Gew.% der kombinierten Menge des hydrophilen Polymers/der mehreren hydrophilen Polymere und des einen niedermolekulargewichtigen Gerüsts oder der mehreren niedermolekulargewichtigen Gerüste stellen;
wobei die Beschichtungszusammensetzung mit dem vorgefertigten geformten Artikel und dem thermoplastischen Substratpolymer (co)extrudiert oder spritzgegossen ist; und wobei das kovalente Vernetzen der Beschichtungszusammensetzung das Ergebnis der Anwesenheit von einem oder mehreren Photoinitiatoren in der Beschichtungszusammensetzung ist, wobei die Photoinitiatoranteile kovalent mit dem niedermolekulargewichtigen Gerüst verknüpft und/oder kovalent in das Grundgerüst des niedermolekulargewichtigen Gerüsts eingebaut sind, und UV- oder sichtbares Licht auf die Beschichtungszusammensetzung einwirken gelassen wird;
wobei der vorgefertigte geformte Artikel und/oder das thermoplastische Substratpolymer ein Polymer ausgewählt aus der folgenden Gruppe umfasst:
Ethylen-(Meth)acrylsäure-Copolymer
Ethylen-(Meth)acrylsäure-Acrylester-Terpolymer
Ethylen-(Meth)acrylsäure-Ionomer und
wobei das medizinische Vorrichtungselement ausgewählt ist aus Rohren und Kathetern.

16. Medizinische Vorrichtung nach einem der Ansprüche 13 bis 15, wobei das medizinische Vorrichtungselement ein Katheter ist.

17. Medizinische Vorrichtung nach einem der Ansprüche 13 bis 15, wobei das medizinische Vorrichtungselement ein Blasenkatheter ist.

## Revendications

1. Procédé de préparation d'un élément de dispositif médical, ledit procédé comprenant les étapes de :
(i) fourniture d'un article formé préfabriqué et/ou une ou plusieurs couches d'un polymère de substrat thermoplastique ;
(ii) fourniture d'une composition de revêtement comprenant :
(a) en tant que seul(s) constituant(s), un ou plusieurs polymère(s) hydrophile(s), et
(b) un ou plusieurs échafaudages de poids moléculaire faible ayant une pluralité de fragments photo-initiateurs de façon covalente liés à ceux-ci et/ou de incorporés de façon covalente dans ceux-ci, dans lequel les fragments photo-initiateurs constituent 0,01 à 20 % en poids de la quantité combinée du ou des polymère(s) hydrophile(s) et des un ou plusieurs échafaudages de poids moléculaire faible ;
(iii) l'extrusion, le moulage par injection ou l'enrobage de poudre de la composition de revêtement de l'étape (ii) sur l'article formé préfabriqué et/ou le polymère de substrat thermoplastique de l'étape (i) de manière à produire l'élément de dispositif médical dudit article formé préfabriqué et/ou ledit polymère de substrat ayant sur celui-ci une couche de ladite composition de revêtement, dans lequel, lorsque ledit article formé préfabriqué et ledit polymère de substrat sont tous deux présents, ledit article formé préfabriqué comporte sur celui-ci une couche dudit polymère de substrat ;
(iv) irradiation de la composition de revêtement avec une lumière UV ou visible de manière à réticuler de façon covalente ladite composition de revêtement ;
dans lequel l'article formé préfabriqué et/ou un ou plusieurs des polymères de substrat thermoplastiques de l'étape (i) comprenant un polymère choisi dans le groupe de :
un copolymère d'éthylène-acide (méth)acrylique
un terpolymère d'éthylène - acide (méth)acrylique-ester acrylique
un ionomère d'éthylène-acide (méth)acrylique ; et
dans lequel l'élément de dispositif médical est choisi parmi des tubes et des cathéters.

2. Procédé selon la revendication 1, dans lequel le polymère hydrophile est un poly(oxyde d'éthylène).

3. Procédé selon l'une quelconque des revendications précédentes, dans lequel l'échafaudage est choisi parmi des polyéthylène glycols, des poly(styrène-co-anhydride maléique)s, des (polyéther aliphatique)-uréthanes, des polyétheramines et des polyesters.

4. Procédé selon l'une quelconque des revendications précédentes, dans lequel le poids moléculaire moyen en poids de l'échafaudage est dans la plage de 100 à 10 000 Da (g/mol).

5. Procédé selon l'une quelconque des revendications précédentes, dans lequel la composition de revêtement est constituée de
20 à 99,99 % en poids des un ou plusieurs polymère (s) hydrophile(s),
0 à 10 % en poids d'un ou plusieurs plastifiants,
0,01 à 80 % en poids des un ou plusieurs échafaudages
de poids moléculaire faible, et 0 à 5 % en poids d'autres composants.

6. Procédé selon l'une quelconque des revendications précédentes, dans lequel un article formé préfabriqué est fourni dans l'étape (i), et dans lequel l'étape (iii) met en oeuvre l'extrusion, le moulage par injection ou l'enrobage de poudre de la composition de revêtement de l'étape (ii) sur l'article formé préfabriqué de l'étape (i) de manière à produire l'élément de dispositif médical dudit article formé préfabriqué ayant sur celui-ci une couche de ladite composition de revêtement.

7. Procédé selon l'une quelconque des revendications 1 à 5, dans lequel un polymère de substrat thermoplastique est fourni dans l'étape (i), et dans lequel l'étape (iii) met en oeuvre l'extrusion ou le moulage par injection de la composition de revêtement de l'étape (ii) conjointement avec le polymère de substrat thermoplastique de l'étape (i) de manière à produire l'élément de dispositif médical dudit polymère de substrat thermoplastique ayant sur celui-ci une couche de ladite composition de revêtement.

8. Procédé selon l'une quelconque des revendications 1 à 5, dans lequel un article formé préfabriqué et un polymère de substrat thermoplastique sont fournis dans l'étape (i), et dans lequel l'étape (iii) met en oeuvre l'extrusion, le moulage par injection ou l'enrobage de poudre de la composition de revêtement de l'étape (ii) sur l'article formé préfabriqué conjointement avec le polymère de substrat thermoplastique de l'étape (i) de manière à produire l'élément de dispositif médical dudit article formé préfabriqué et ledit polymère de substrat thermoplastique, ledit article formé préfabriqué ayant sur celui-ci une couche dudit polymère de substrat thermoplastique et ledit polymère de substrat thermoplastique ayant sur celui-ci une couche de ladite composition de revêtement.

9. Procédé selon l'une quelconque des revendications 1 à 5 et 7 à 8, dans lequel l'élément de dispositif médical comprend une couche d'un polymère de substrat thermoplastique.

10. Procédé selon l'une quelconque des revendications 1 à 5 et 7 à 8, dans lequel l'élément de dispositif médical comprend deux couches d'un polymère de substrat thermoplastique.

11. Procédé selon l'une quelconque des revendications précédentes, dans lequel l'élément de dispositif médical est un cathéter.

12. Procédé selon l'une quelconque des revendications précédentes, dans lequel l'élément de dispositif médical est un cathéter urinaire.

13. Dispositif médical comprenant un élément de dispositif médical d'un polymère de substrat thermoplastique ayant sur celui-ci une couche d'une composition de revêtement réticulée de façon covalente de
(a) en tant que seul(s) constituant(s), un ou plusieurs polymère(s) hydrophile(s), et
(b) un ou plusieurs échafaudages de poids moléculaire faible ayant une pluralité de résidus de fragments photo-initiateurs, les résidus de fragments photo-initiateurs constituant 0,01 à 20 % en poids de la quantité combinée des un ou plusieurs polymère(s) hydrophile(s) et des un ou plusieurs échafaudages de poids moléculaire faible ;
dans lequel ladite composition de revêtement est (co)extrudée ou moulée par injection avec ledit polymère de substrat thermoplastique ; et dans lequel la réticulation covalente de la composition de revêtement est la conséquence de la présence d'un ou plusieurs photo-initiateurs dans la composition de revêtement, lesdits fragments photo-initiateurs étant liés de façon covalente à l'échafaudage de poids moléculaire faible et/ou étant incorporés de façon covalente dans le squelette de l'échafaudage de poids moléculaire faible, et l'exposition de la composition de revêtement à une lumière UV ou visible ; le polymère de substrat thermoplastique comprenant un polymère choisi dans le groupe de :
un copolymère d'éthylène-acide (méth)acrylique
un terpolymère d'éthylène - acide (méth)acrylique-ester acrylique
un ionomère d'éthylène-acide (méth)acrylique ; et
dans lequel l'élément de dispositif médical est choisi parmi des tubes et des cathéters.

14. Dispositif médical comprenant un élément de dispositif médical d'un article formé préfabriqué ayant sur celui-ci une couche d'une composition de revêtement réticulée de façon covalente de
(a) en tant que seul(s) constituant(s), un ou plusieurs polymère(s) hydrophile(s), et
(b) un ou plusieurs échafaudages de poids moléculaire faible ayant une pluralité de résidus de fragments photo-initiateurs, les résidus de fragments photo-initiateurs constituant 0,01 à 20 % en poids de la quantité combinée des un ou plusieurs polymère(s) hydrophile(s) et des un ou plusieurs échafaudages de poids moléculaire faible ;
dans lequel ladite composition de revêtement est extrudée ou moulée par injection avec ledit article formé préfabriqué ; et dans lequel la réticulation covalente de la composition de revêtement est la conséquence de la présence d'un ou plusieurs photo-initiateurs dans la composition de revêtement, lesdits fragments photo-initiateurs étant liés de façon covalente à l'échafaudage de poids moléculaire faible et/ou étant incorporés de façon covalente dans le squelette de l'échafaudage de poids moléculaire faible, et l'exposition de la composition de revêtement à une lumière UV ou visible ;
dans lequel l'article formé préfabriqué comprend un polymère choisi dans le groupe de :
un copolymère d'éthylène-acide (méth)acrylique
un terpolymère d'éthylène - acide (méth)acrylique-ester acrylique
un ionomère d'éthylène-acide (méth)acrylique ; et
dans lequel l'élément de dispositif médical est choisi parmi des tubes et des cathéters.

15. Dispositif médical comprenant un élément de dispositif médical d'un article formé préfabriqué ayant sur celui-ci une couche d'un polymère de substrat thermoplastique, où ledit polymère de substrat thermoplastique comporte sur celui-ci une couche d'une composition de revêtement réticulée de façon covalente de
(a) en tant que seul(s) constituant(s), un ou plusieurs polymère(s) hydrophile(s), et
(b) un ou plusieurs échafaudages de poids moléculaire faible ayant une pluralité de résidus de fragments photo-initiateurs, les résidus de fragments photo-initiateurs constituant 0,01 à 20 % en poids de la quantité combinée des un ou plusieurs polymère(s) hydrophile(s) et des un ou plusieurs échafaudages de poids moléculaire faible ;
dans lequel ladite composition de revêtement est (co)extrudée ou moulée par injection avec ledit article formé préfabriqué et ledit polymère de substrat thermoplastique ; et dans lequel la réticulation covalente de la composition de revêtement est la conséquence de la présence d'un ou plusieurs photo-initiateurs dans la composition de revêtement, lesdits fragments photo-initiateurs étant liés de façon covalente à l'échafaudage de poids moléculaire faible et/ou étant incorporés de façon covalente dans le squelette de l'échafaudage de poids moléculaire faible, et l'exposition de la composition de revêtement à une lumière UV ou visible ;
dans lequel l'article formé préfabriqué et/ou le polymère de substrat thermoplastique comprend un polymère choisi dans le groupe de :
un copolymère d'éthylène-acide (méth)acrylique
un terpolymère d'éthylène - acide (méth)acrylique-ester acrylique
un ionomère d'éthylène-acide (méth)acrylique ; et
dans lequel l'élément de dispositif médical est choisi parmi des tubes et des cathéters.

16. Dispositif médical selon l'une quelconque des revendications 13 à 15, dans lequel l'élément de dispositif médical est un cathéter.

17. Dispositif médical selon l'une quelconque des revendications 13 à 15, dans lequel l'élément de dispositif médical est un cathéter urinaire.
